# EUROPÄISCHE PATENTSCHRIFT

(11) **EP 1 525 202 B1**
(45) Veröffentlichungstag und Bekanntmachung des Hinweises auf die Patenterteilung: **11.01.2006**
(21) Anmeldenummer: 03764943.1
(22) Anmeldetag: 07.07.2003
(51) Int. Cl.: C07D 471/04, A61K 31/506, A61P 25/00

(54) **NEUE 2,5-DISUBSTITUIERTE PYRIMIDINDERIVATE**
NOVEL 2,5-DISUBSTITUTED PYRIMIDINE DERIVATIVES
NOUVEAUX DERIVES DE PYRIMIDINE 2,5-DISUBSTITUES

(30) Priorität: 18.07.2002 DE 10232572
(43) Veröffentlichungstag der Anmeldung: 27.04.2005
(73) Patentinhaber: Bayer HealthCare AG, 51368 Leverkusen (DE)
(72) Erfinder: FEURER, Achim, 69259 Wilhelmsfeld (DE); LUITHLE, Joachim, 42489 Wülfrath (DE); WIRTZ, Stephan-Nicholas, 42327 Wuppertal (DE); KÖNIG, Gerhard, Arlington, MA 02476 (US); STASCH, Johannes-Peter, 42651 Solingen (DE); STAHL, Elke, 51467 Bergisch Gladbach (DE); SCHREIBER, Rudy, Menlo Park, CA 94025 (US); WUNDER, Frank, 42117 Wuppertal (DE); LANG, Dieter, 42553 Velbert (DE)
(86) Internationale Anmeldenummer: PCT/EP2003/007238
(87) Internationale Veröffentlichungsnummer: WO 2004/009589

(56) Entgegenhaltungen:
- DE-A- 19 649 460
- DE-A- 19 834 045
- DE-A- 19 834 047
- DE-A- 19 846 514

## Beschreibung

Die vorliegende Erfindung betrifft neue 2,5-disubstituierte Pyrimidinderivate, welche die lösliche Guanylatcyclase stimulieren, Verfahren zu ihrer Herstellung sowie ihre Verwendung zur Herstellung von Arzneimitteln, insbesondere von Arzneimitteln zur Behandlung von Krankheiten des Zentralnervensystems.

Eines der wichtigsten zellulären Signalübertragungssysteme in Säugerzellen ist das cyclische Guanosinmonophosphat (cGMP). Zusammen mit Stickstoffmonoxid (NO), das aus dem Endothel freigesetzt wird und hormonelle und mechanische Signale überträgt, bildet es das NO/cGMP-System. Die Guanylatcyclasen katalysieren die Biosynthese von cGMP aus Guanosintriposphat (GTP). Die bisher bekannten Vertreter dieser Familie lassen sich sowohl nach strukturellen Merkmalen als auch nach der Art der Liganden in zwei Gruppen aufteilen: Die partikulären, durch natriuretische Peptide stimulierbaren Guanylatcyclasen und die löslichen, durch NO stimulierbaren Guanylatcyclasen. Die löslichen Guanylatcyclasen bestehen aus zwei Untereinheiten und enthalten mindestens ein Häm pro Heterodimer. Die Hämgruppen sind Teil des regulatorischen Zentrums und haben eine zentrale Bedeutung für den Aktivierungsmechanismus. NO kann an das Eisenatom des Häms gebunden werden und so die Aktivität des Enzyms deutlich erhöhen. Hämfreie Präparationen lassen sich hingegen nicht durch NO stimulieren. Auch CO kann am Eisen-Zentralatom des Häms gebunden werden, wobei die Stimulierung durch CO deutlich geringer als die durch NO ist.

Durch die Bildung von cGMP und der daraus resultierenden Regulation von Phosphodiesterasen, Ionenkanälen und Proteinkinasen spielt die Guanylatcyclase eine entscheidende Rolle bei unterschiedlichen physiologischen Prozessen, insbesondere bei der Relaxation und Proliferation glatter Muskelzellen, der Plättchenaggregation und -adhäsion und der neuronalen Signalübertragung sowie bei Erkrankungen, welche auf einer Störung der vorstehend genannten Vorgänge beruhen. Unter pathophysiologischen Bedingungen kann das NO/cGMP-System supprimiert sein. Bei Alzheimer Patienten beispielsweise ist die NO-stimulierte Aktivität der löslichen Guanylatcyclase im Gehirn (cortex cerebralis) stark reduziert.

Durch Applikation von Dizocilpine, welches zu einem reduzierten cGMP-Spiegel führt, kann ein reduziertes Lemverhalten bei Versuchstieren beobachtet werden (Yamada et al., Neuroscience 74 (1996), 365-374). Diese Beeinträchtigung kann durch Injektion von 8-Br-cGMP, einer membrangängigen Form von cGMP, aufgehoben werden. Dies steht im Einklang mit Untersuchungen, die zeigen, dass sich der cGMP-Spiegel im Gehim nach Lern- und Gedächtnisaufgaben erhöht.

Eine auf die Beeinflussung des cGMP-Signalweges in Organismen abzielende NOunabhängige Behandlungsmöglichkeit ist aufgrund der zu erwartenden hohen Effizienz und geringen Nebenwirkungen ein vielversprechender Ansatz für die Stimulation der löslichen Guanylatcyclase.

Zur therapeutischen Stimulation der löslichen Guanylatcyclase wurden bisher ausschließlich Verbindungen wie organische Nitrate verwendet, deren Wirkung auf der Freisetzung von NO beruht. Dieses wird durch Biokonversion gebildet und aktiviert die lösliche Guanylatcyclase durch Bindung am Eisenzentralatom des Häms. Neben den Nebenwirkungen gehört die Toleranzentwicklung zu den entscheidenden Nachteilen dieser Behandlungsweise.

In den letzten Jahren wurden Substanzen beschrieben, die die lösliche Guanylatcyclase direkt, d.h. ohne vorherige Freisetzung von NO, stimulieren, wie beispielsweise 3-(5'-Hydroxymethyl-2'-furyl)-1-benzylindazol (YC-1, Wu et al., Blood δ4 (1994), 4226; Mülsch et al., Br. J. Pharmacol. 120 (1997), 681), Fettsäuren (Goldberg et al., J. Biol. Chem. 252 (1977), 1279), Diphenyliodonium-hexafluorophosphat (Pettibone et al., Eur. J. Pharmacol. 116 (1985), 307), Isoliquiritigenin (Yu et al., Brit. J. Pharmacol. 114 (1995), 1587) sowie verschiedene substituierte Pyrazolderivate (WO 9S/16223).

Weiterhin sind in der WO 98/16507, WO 98/23619, WO 00/06567, WO 00/06568, WO 00/06569, WO 00/21954, WO 02/4229, WO 02/4300, WO 02/4301 und WO 02/4302 Pyrazolopyridinderivate als Stimulatoren der löslichen Guanylatcyclase beschrieben. In diesen Patentanmeldungen sind auch Pyrazolopyridine beschrieben, welche unterschiedliche Reste aufweisen. Derartige Verbindungen weisen eine sehr hohe in-vitro-Aktivität bezüglich der Stimulation der löslichen Guanylatcyclase auf. Allerdings zeigte sich, dass diese Verbindungen hinsichtlich ihrer in vivo-Eigenschaften, wie beispielsweise ihres Verhaltens in der Leber, ihres pharmakokinetischen Verhaltens, ihrer Dosis-Wirkungsbeziehung und ihres Metabolisierungswegs einige Nachteile aufweisen.

Es war daher die Aufgabe der vorliegenden Erfindung, weitere Pyrimidinderivate bereitzustellen, welche als Stimulatoren der löslichen Guanylatcyclase wirken, aber nicht die vorstehend aufgeführten Nachteile der Verbindungen aus dem Stand der Technik aufweisen. Ein zusätzlicher Vorteil neuer Arzneimittel zur Behandlung von Krankheiten im Zentralnervensystem (z.B. Lern- und Gedächtnisstörungen) wäre eine erhöhte Selektivität gegenüber peripheren kardiovaskulären Wirkungen. Diese sollte ebenfalls (z.B. durch bessere Hirngängigkeit) gegenüber dem Stand der Technik verbessert werden.

Diese Aufgabe wird gemäß der vorliegenden Erfindung durch die Verbindungen gemäß Anspruch 1 gelöst.

Im einzelnen betrifft die vorliegende Erfindung die Verbindungen der Formel 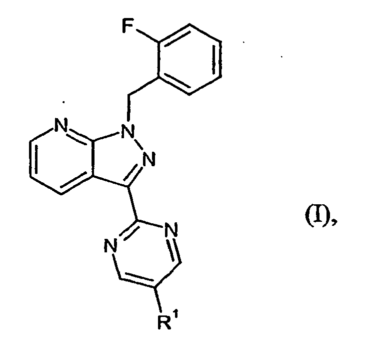
in welcher
- R¹: C₆-C₁₀-Aryl oder 5- bis 10-gliedriges Heteroaryl, die gegebenenfalls durch Reste ausgewählt aus der Gruppe Halogen, Cyano, C₁-C₆-Alkoxy, C₁-C₆₋Alkoxycarbonyl, Trifluormethyl, 2,2,2-Trifluorethyl, Trifluormethoxy, C₁-C₄₋Alkyl und C₃-C₈-Cycloalkyl substituiert sind, wobei C₁-C₄-Alkyl gegebenenfalls mit Hydroxy substituiert ist,
oder eine Gruppe der Formel 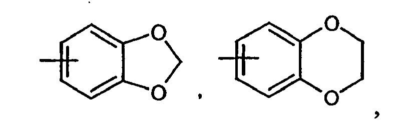
oder
über ein Stickstoffatom gebundenes 4- bis 12-gliedriges Heterocyclyl, das gegebenenfalls durch Reste ausgewählt aus der Gruppe -NHR², Halogen, C₁₋C₆-Alkoxycarbonyl, C₁-C₆-Alkoxy, C₁-C₆-Alkyl und Oxo substituiert ist, wobei C₁-C₆-Alkyl gegebenenfalls mit Hydroxy substituiert ist, und
R² C₁-C₄-Alkyl,
oder
C₄-C₈-Cycloalkyl, das in der der Anknüpfungsstelle benachbarten Position durch Oxo substituiert ist und das gegebenenfalls durch C₁-C₄-Alkyl substituiert ist, bedeuten
und deren Salze, Solvate und/oder Solvate der Salze.

Sofern in R¹ asymmetrische C-Atome enthalten sind, können die erfindungsgemäßen Verbindungen als Enantiomere, Diastereomere oder Mischungen aus diesen vorliegen. Diese Mischungen lassen sich in bekannter Weise in die stereoisomer einheitlichen Bestandteile trennen.

Als Salze sind im Rahmen der Erfindung physiologisch unbedenkliche Salze der erfindungsgemäßen Verbindungen bevorzugt.

Physiologisch unbedenkliche Salze der erfindungsgemäßen Verbindungen können Säureadditionssalze der Verbindungen mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Physiologisch unbedenkliche Salze können auch Salze mit üblichen Basen sein, wie beispielsweise Alkalimetallsalze (z.B. Natrium- oder Kaliumsalze), Erdalkalisalze (z.B. Calcium- oder Magnesiumsalze) oder Ammoniumsalze, abgeleitet von Ammoniak oder organischen Aminen wie beispielsweise Diethylamin, Triethylamin, Ethyldiisopropylamin, Prokain, Dibenzylamin, N-Methylmorpholin, Dihydroabiethylamin, 1-Ephenamin oder Methyl-piperidin.

Solvate der erfindungsgemäßen Verbindungen sind im Rahmen der Erfindung stöchiometrische Zusammensetzungen der Verbindungen oder ihrer Salze mit Lösungsmittel, z.B. Wasser, Ethanol.

Im Rahmen der vorliegenden Erfindung haben die Substituenten im Allgemeinen die folgende Bedeutung:
C₁-C₆-Alkyl steht für einen geradkettigen oder verzweigten Alkylrest mit 1 bis 6 Kohlenstoffatomen. Bevorzugt ist ein geradkettiger oder verzweigter Alkylrest mit 1 bis 4, besonders bevorzugt mit 1 bis 3 Kohlenstoffatomen. Nicht-limitierende Beispiele umfassen Methyl, Ethyl, n-Propyl, Isopropyl, tert.Butyl, n-Pentyl und n-Hexyl.
C₁-C₆-Alkoxy steht für einen geradkettigen oder verzweigten Alkoxyrest mit 1 bis 6 Kohlenstoffatomen. Bevorzugt ist ein geradkettiger oder verzweigter Alkoxyrest mit 1 bis 4, besonders bevorzugt mit 1 bis 3 Kohlenstoffatomen. Nicht-limitierende Beispiele umfassen Methoxy, Ethoxy, n-Propoxy, Isopropoxy, tert.Butoxy, n-Pentoay und n-Hexoxy.
C₁-C₆-Alkoxycarbonyl steht für einen geradkettigen oder verzweigten Alkoxycarbonylrest mit 1 bis 6 Kohlenstoffatomen. Bevorzugt ist ein geradkettiger oder verzweigter Alkoxycarbonylrest mit 1 bis 4, besonders bevorzugt mit 1 bis 3 Kohlenstoffatomen. Nicht-limitierende Beispiele umfassen Methoxycarbonyl, Ethoxycarbonyl, n-Propoxycarbonyl, Isopropoxycarbonyl und tert.Butoxycarbonyl.
C₆-C₁₀-Aryl steht für einen aromatischen Rest mit 6 bis 10 Kohlenstoffatomen. Nicht-limitierende Beispiele umfassen Phenyl und Naphthyl.
C₃-C₈-Cycloalkyl steht für Cyclopropyl, Cyclopentyl, Cyclobutyl, Cyclohexyl, Cycloheptyl oder Cyclooctyl. Nicht-limitierende Beispiele umfassen Cyclopropyl, Cyclopentyl und Cyclohexyl.
Halogen steht für Fluor, Chlor, Brom und Jod. Bevorzugt sind Fluor, Chlor und Brom. Besonders bevorzugt sind Fluor und Chlor.
5- bis 10-gliedriges Heteroaryl steht für einen aromatischen; mono- oder bicyclischen Rest mit 5 bis 10 Ringatomen und bis zu 5 Heteroatomen aus der Reihe S, O und/oder N. Bevorzugt sind 5- bis 6-gliedrige Heteroaryle mit bis zu 4 Heteroatomen. Der Heteroarylrest kann über ein Kohlenstoff- oder Stickstoffatom gebunden sein. Nicht-limitierende Beispiele umfassen Thienyl, Furyl, Pyrrolyl, Thiazolyl, Oxazolyl, Imidazolyl, Pyridyl, Pyrimidyl, Pyridazinyl, Indolyl, Indazolyl, Isoxazyl, Benzofuranyl, Benzothiophenyl, Chinolinyl, Isochinolinyl.
4- bis 12-gliedriges Heterocyclyl steht für einen mono- oder polycyclischen heterocyclischen Rest mit 4 bis 12 Ringatomen und bis zu 3, vorzugsweise 2 Heteroatomen bzw. Heterogruppen aus der Reihe N, O, S, SO, SO₂. 4- bis 8-gliedriges Heterocyclyl ist bevorzugt. Mono- oder bicyclisches Heterocyclyl ist bevorzugt. Als Heteroatome sind N und O bevorzugt. Die Heterocyclyl-Reste können gesättigt oder teilweise ungesättigt sein. Gesättigte Heterocyclyl-Reste sind bevorzugt. Nicht-limitierende Beispiele umfassen Oxetan-3-yl, Pyrrolidin-2-yl, Pyrrolidin-3-yl, Pyrrolinyl, Tetrahydrofuranyl, Tetrahydrothienyl, Pyranyl, Piperidinyl, Thiopyranyl, Morpholinyl, Perhydroazepinyl, 9-Oxa-3,7-diazabicyclo[3.3.1]nonanyl, 1-Oxa-4,7-diazaspiro[5.4]-decanyl, 10-Oxa-4-azatricyclo[5.2.1.0^{2,6}]decanyl, 10-Oxa-4-azatricyclo[5.2.1.0^{2,6}]-decanyl, Decahydropyrrolo[3,4-b]pyrrolizidinyl, 2,5-Diazabicyclo[2.2.1]heptanyl, 8-oxa-3-azabicyclo[3.2.1]octanyl, Octahydropyrrolo[3,4-d][1,3]oxazinyl.

Sofern die Reste in den erfindungsgemäßen Verbindungen substituiert sind, können die Reste, soweit nicht anders spezifiziert, ein- oder mehrfach gleich oder verschieden substituiert sein. Eine Substitution mit bis zu drei gleichen oder verschiedenen Substituenten ist bevorzugt. Ganz besonders bevorzugt ist die Substitution mit einem Substituenten.

Ganz besonders bevorzugt sind Kombinationen von zwei oder mehreren der oben genannten Vorzugsbereiche.

Eine weitere Ausführungsform der Erfindung betrifft Verbindungen der Formel (I),
wobei
- R¹: Phenyl oder 5- bis 6-gliedriges Heteroaryl, die gegebenenfalls durch Reste ausgewählt aus der Gruppe Fluor, Chlor, Cyano, C₁-C₃-Alkoxycarbonyl, C₁₋C₃-Akoxy, Trifluormethyl, 2,2,2-Trifluorethyl, Trifluormethoxy, C₁-C₃₋Alkyl, und C₃-C₅-Cycloakyl substituiert sind, wobei C₁-C₃-Alkyl gegebenenfalls mit Hydroxy substituiert ist,

oder ein Gruppe der Formel 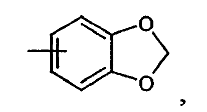
oder
über ein Stickstoffatom gebundenes 4- bis 12-gliedriges Heterocyclyl, das gegebenenfalls durch Reste ausgewählt aus der Gruppe -NHR², Fluor, Chlor, C₁-C₃-Alkyl, C₁-C₃-Akoxycarbonyl, C₁-C₃-Alkoxy und Oxo substituiert ist, wobei C₁-C₃-Alkyl gegebenenfalls mit Hydroxy substituiert ist und
R² C₁-C₃-Alkyl,
oder
Cyclohexyl, das in der der Anknüpfungsstelle benachbarten Position durch Oxo substituiert ist und das gegebenenfalls durch C₁-C₂-Alkyl substituiert ist, bedeuten
und deren Salze, Solvate und/oder Solvate der Salze.

Eine weiter Ausführungsform der Erfindung betrifft Verbindungen der Formel (I)
wobei
- R¹: Phenyl oder Pyridyl, Pyrazolyl, Isoxazolyl, die gegebenenfalls durch Reste ausgewählt aus der Gruppe Fluor, Chlor, Cyano, Methoxy, Methoxycarbonyl, Ethoxycarbonyl, Trifluormethyl, 2,2,2-Trifluorethyl, Trifluormethoxy, Methyl, Cyclopropyl oder Hydroxymethyl substituiert sind

oder eine Gruppe der Formel 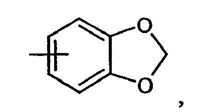
oder
über ein Stickstoffatom gebundenes 4- bis 12-gliedriges Heterocyclyl, das gegebenenfalls durch Reste ausgewählt aus der Gruppe -NHR², Fluor, Chlor, C₁-C₃-Alkyl, Methoxy, Ethoxy, Hydroxymehtyl und Oxo substituiert ist, und
R² Methyl,
oder
Cyclohexyl, das in der der Anknüpfungsstelle benachbarten Position durch Oxo substituiert ist, und das gegebenenfalls durch Methyl substituiert ist, bedeuten
und deren Salze, Solvate und/oder Solvate der Salze.

Die Erfindung betrifft weiterhin Verfahren zur Herstellung der erfindungsgemäßen Verbindungen, wonach man entweder
[A] Verbindungen der Formel 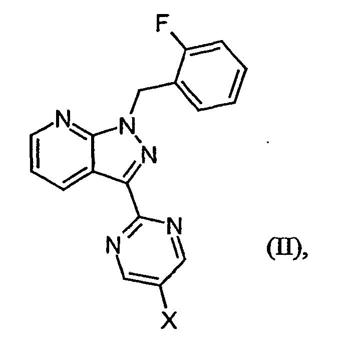
   in welcher X Chlor, Brom, lod, bevorzugt Brom bedeutet, mit einer Verbindung der Formel (III),

   R³-NH-R⁴ (III),

   in welcher
   - R³, R⁴: zusammen mit dem Stickstoffatom, an das sie gebunden sind, 4- bis 12-gliedriges Heterocyclyl, das gegebenenfalls durch Reste ausgewählt aus der Gruppe -NHR², Halogen, C₁-C₆-Alkoxycarbonyl, C₁-C₆-Alkoxy, C₁-C₆₋Alkyl und Oxo substituiert ist, wobei C₁-C₆-Alkyl gegebenenfalls mit -OR⁵ substituiert ist, und R² die oben angegebene Bedeutung hat, R⁵ eine Hydroxy-Schutzgruppe, bevorzugt Tri-(C₁-C₆-alkyl)silyl bedeutet, in einem inerten Lösungsmittel in Gegenwart einer Base und eines Übergangsmetallkatalysators zu Verbindungen der Formel

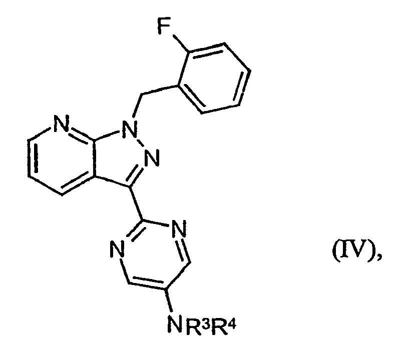

   umsetzt,
   oder
[B] Verbindungen der Formel (II) mit einer Verbindung der Formel 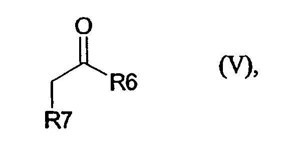
   in welcher
   - R⁶: Cycloalkyl, R⁷ Wasserstoff oder R⁶ und R⁷ zusammen mit der CH₂CO-Gruppe, an die sie gebunden sind, Cycloalkyl, das durch C₁-C₆-Alkylreste substituiert sein kann, bedeuten, in einem inerten Lösungsmittel in Gegenwart einer Base und eines Übergangsmetallkatalysators zu Verbindungen der Formel

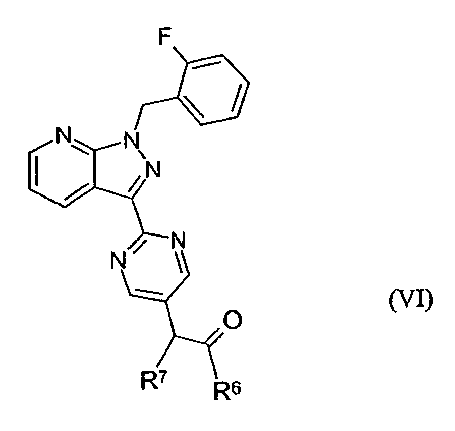

   umsetzt,
   oder
[C] Verbindungen der Formel (II) mit einer Verbindung der Formel

   A-R⁸ (VII),

   in welcher
   - A: -B(OR⁹)₂ oder -Sn(C₁-C₆-Alkyl)₃, wobei
   R⁹ Wasserstoff, C₁-C₆-Alkyl oder beide Reste zusammen eine -CH₂CH₂- oder -(CH₃)₂C-C(CH₃)₂- Brücke bilden
   und
   - R⁸: C₆-C₁₀-Aryl oder 5- bis 10-gliedriges Heteroaryl, die gegebenenfalls durch Reste ausgewählt aus der Gruppe Halogen, Cyano, C₁-C₆-Alkoxy, C₁-C₆₋Alkoxycarbonyl, Trifluormethyl, 2,2,2-Trifluorethyl, Trifluormethoxy, C₁-C₄₋Alkyl und C₃-C₈-Cycloalkyl substituiert sind, wobei C₁-C₄-Alkyl gegebenenfalls mit Hydroxy substituiert ist, oder eine Gruppe der Formel 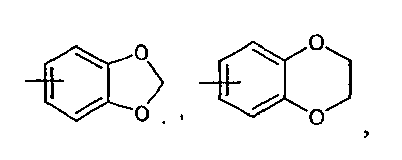
   bedeutet, in einem inerten Lösungsmittel in Gegenwart einer Base und eines
   Übergangsmetallkatalysators zu Verbindungen der Formel

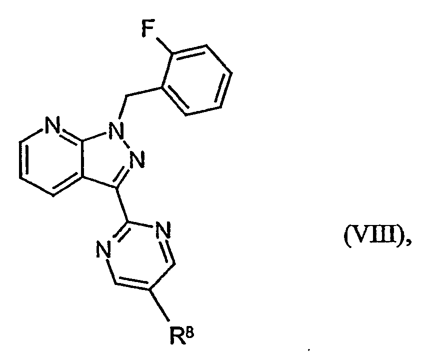

   umsetzt,
   und die resultierenden Verbindungen der Formel (I), (IV), (VI) und (VIII) gegebenenfalls mit den entsprechenden (i) Lösungsmitteln und/oder (ii) Basen oder Säuren zu ihren Solvaten, Salzen oder Solvaten der Salze umsetzt.

Vorzugsweise werden die erfindungsgemäßen Verfahren [A] und [B] in einem Temperaturbereich von 20 bis 100 °C und das erfindungsgemäße Verfahren [C] von 20 bis 150 °C bei Normaldruck durchgeführt.

Inerte Lösungsmittel sind beispielsweise Ether wie Dioxan, Tetrahydrofuran oder 1,2-Dimethoxyethan, Kohlenwasserstoffe wie Benzol, Xylol oder Toluol, Nitroaromaten wie Nitrobenzol, gegebenenfalls *N*-alkylierte Carbonsäureamide wie Dimethylformamid, Dimethylacetamid, Alkylsulfoxide wie Dimethylsulfoxid oder Lactame wie *N*-Methylpyrrolidon. Bevorzugt sind Lösungsmittel aus der Reihe Dimethylformamid, 1,2-Dimethoxyethan, Toluol und Dioxan.

Basen sind beispielsweise Alkalialkoholate wie beispielsweise Natrium- oder Kalium-tert-butylat oder Akalicarbonate wie Cäsiumcarbonat, Natrium- oder Kaliumcarbonat oder Alkalihydride wie Natrium- oder Kaliumhydrid.

Übergangsmetallkatalysatoren können bevorzugt Palladium(0)- oder Palladium- (II)-verbindungen sein, die präformiert eingesetzt wie beispielsweise Bis-(diphenylphosphanferrocenyl)-palladium(II)-chlorid, Dichlorbis(triphenylphosphin)-palladium oder in situ aus einer geeigneten Palladiumquelle wie beispielsweise Bis(dibenzylidenaceton)-palladium(0) oder Tetrakis-triphenylphosphin-palladium(0) und einem geeigneten Phosphinligand erzeugt werden können. Besonders bevorzugt ist der Einsatz von 2,2'-Bis-(diphenylphosphino)-1,1'-binaphthalin (BINAP) als Phosphinligand.

Die übergangsmetallkatalysierten Reaktionen können analog literaturbekannten Verfahren durchgeführt werden, z. B. Umsetzung mit Alkinen: vgl. N. Krause et al., *J. Org. Chem.* 1998, 63, 8551; mit Ketonen, Aromaten und Alkenen: vgl. z.B. A. Suzuki, *Acc. Chem. Res.* 1982, 15, 178ff; Miyaura et al. *J. Am. Chem. Soc*. 1989, *111,* 314; J. K. Stille, *Angew. Chem.* 1986, 98, 504 und mit substituierten Aminen: vgl. S. L. Buchwald et al., *J. Organomet. Chem.* 1999, 576, 125ff. (siehe auch J. Tsuji, Palladium Reagents and Catalysts, Wiley, New York, 1995).

Das erfindungsgemäße Verfahren kann durch das folgende Syntheseschema verdeutlicht werden.

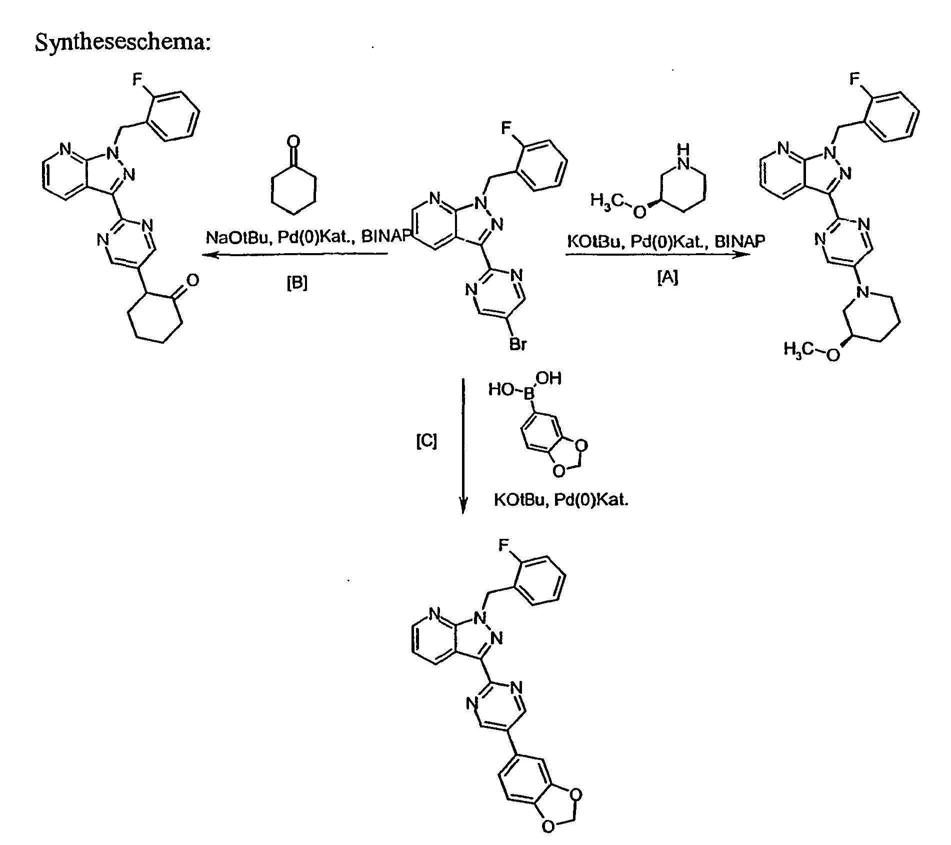

Funktionelle Gruppen können gegebenenfalls mit geeigneten, Schutzgruppen geschützt werden, die anschließend wieder abgespalten werden können (vgl. z. B. T. W. Greene, P. Wuts, "Protective Groups in Organic Synthesis", 2.Aufl., Wiley; New York, 1991).

Die Verbindungen der Formeln (IV), (VI) und (VII) können durch Entschützen funktioneller Gruppen und eine gegebenenfalls darauf folgende Derivatisierung durch bekannte Verfahren zur Alkylierung, Oxidation, Reduktion, Veretherung in die erfindungsgemäßen Verbindungen der Formel (I) überführt werden die gegebenenfalls mit den entsprechenden (i) Lösungsmitteln und/oder (ii) Basen oder Säuren zu ihren Solvaten, Salzen oder Solvaten der Salze umgesetzt werden. Dies soll durch das folgende Syntheseschema anhand eines Beispiels (Entschützung, Alkylierung) verdeutlicht werden.

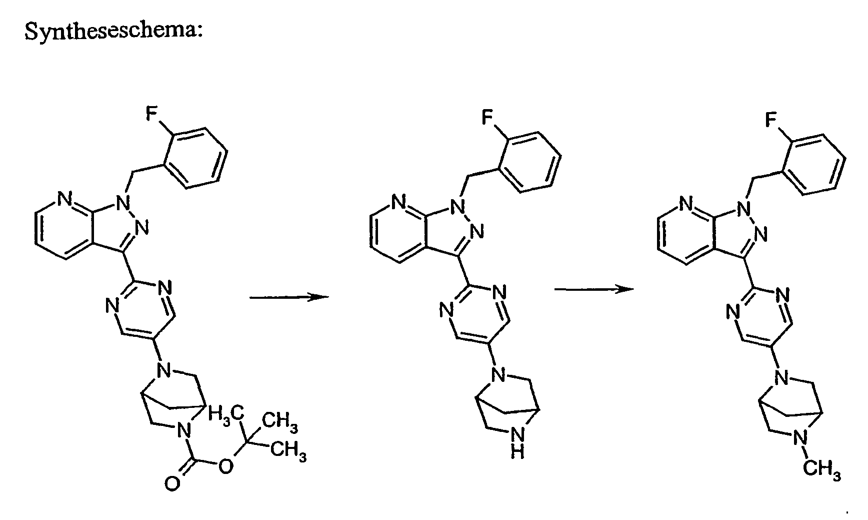

Verbindungen der Formel (II) können durch Umsetzung von Verbindung der Formel 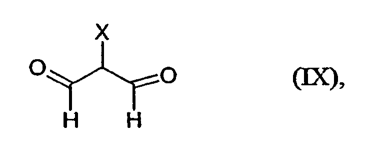
in welcher X die oben angegebene Bedeutung hat,
mit einer Verbindung der Formel 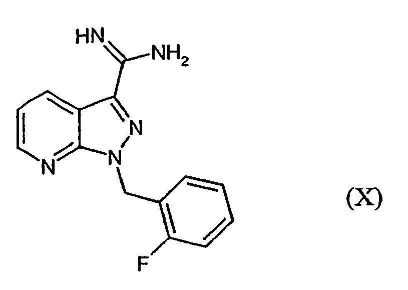 hergestellt werden.

Die Verbindungen der Formel (III), (V), (VII), und (IX) sind kommerziell erhältlich, bekannt oder können nach bekannten Verfahren hergestellt werden.

Die Verbindung der Formel (X) ist aus der WO 00/06569 bekannt.

Die erfindungsgemäßen Verbindungen zeigen ein nicht vorhersehbares, wertvolles pharmakologisches Wirkspektrum.

Die in erfindungsgemäßen Verbindungen erhöhen die cGMP-Spiegel in Neuronen und stellen somit Wirkstoffe zur Bekämpfung von Krankheiten im Zentralnervensystem dar, die durch Störungen des NO/cGMP-Systems gekennzeichnet sind. Insbesondere sind sie geeignet zur Verbesserung der Wahrnehmung, Konzentrationsleistung, Lernleistung, oder Gedächtnisleistung nach kognitiven Störungen, wie sie insbesondere bei Situationen/Krankheiten/Syndromen auftreten wie "Mild cognitive impairment, Altersassoziierte Lern- und Gedächtnisstörungen, Altersassoziierte Gedächtnisverluste, Vaskuläre Demenz, Schädel-Hirn-Trauma, Schlaganfall, Demenz, die nach Schlaganfällen auftritt ("post stroke dementia"), post-traumatisches Schädel Hirn Trauma, allgemeine Konzentrationsstörungen, Konzentrationsstörungen bei Kindern mit Lern- und Gedächtnisproblemen, Alzheimersche Krankheit, Demenz mit Lewy-Körperchen, Demenz mit Degeneration der Frontallappen einschließlich des Pick's Syndroms, Parkinsonsche Krankheit, Progressive nuclear palsy, Demenz mit corticobasaler Degeneration, Amyolateralsklerose (ALS), Huntingtonsche Krankheit, Multiple Sklerose, Thalamische Degeneration, Creutzfeld-Jacob-Demenz, HIV-Demenz, Schizophrenie mit Demenz oder Korsakoff-Psychose.

Die erfindungsgemäßen Verbindungen führen auch zu einer Gefäßrelaxation, Thrombozytenaggregationshemmung und zu einer Blutdrucksenkung sowie zu einer Steigerung des koronaren Blutflusses. Diese Wirkungen sind über eine direkte Stimulation der löslichen Guanylatcyclase und einem intrazellulären cGMP-Anstieg vermittelt. Außerdem können die erfindungsgemäßen Verbindungen die Wirkung von Substanzen, die den cGMP-Spiegel steigern, wie beispielsweise EDRF (Endothelium derived relaxing factor), NO-Donatoren, Protoporphyrin IX, Arachidonsäure oder Phenylhydrazinderivate verstärken.

Sie können daher in Arzneimitteln zur Behandlung von kardiovaskulären Erkrankungen wie beispielsweise zur Behandlung des Bluthochdrucks und der Herzinsuffzienz, stabiler und instabiler Angina pectoris, peripheren und kardialen Gefäßerkrankungen, von Arrhythmien, zur Behandlung von thromboembolischen Erkrankungen und Ischämien wie Myokardinfarkt, Hirnschlag, transistorisch und ischämische Attacken, periphere Durchblutungsstörungen, Verhinderung von Restenosen wie nach Thrombolysetherapien durch beispielsweise der Verwendung in Stents, percutan transluminalen Angioplastien (PTA), percutan transluminalen Koronarangioplastien (PTCA), Bypass-Operationen sowie zur Behandlung von Arteriosklerose, asthmatischen Erkrankungen, Osteoporose, Gastroparese, Glaucom und Krankheiten des Urogenitalsystems wie beispielsweise Inkontinenz, Prostatahypertrophie, erektile Dysfunktion und weibliche sexuelle Dysfunktion eingesetzt werden.

Sie eignen sich auch zur Behandlung von Erkrankungen des Zentralnervensystems wie Angst-, Spannungs- und Depressionszuständen, zentralnervös bedingten Sexualdysfunktionen und Schlafstörungen, sowie zur Regulierung krankhafter Störungen der Nahrungs-, Genuss- und Suchtmittelaufnahme.

Weiterhin eignen sich die erfindungsgemäßen Verbindungen zur Regulation der cerebralen Durchblutung und können wirkungsvolle Mittel zur Bekämpfung von Migräne darstellen.

Auch eignen sie sich zur Prophylaxe und Bekämpfung der Folgen cerebraler Infarktgeschehen (Apoplexia cerebri) wie Schlaganfall, cerebraler Ischämien und des Schädel-Hirn-Traumas. Ebenso können die erfindungsgemäßen Verbindungen zur Bekämpfung von Schmerzzuständen eingesetzt werden.

Zudem besitzen die erfindungsgemäßen Verbindungen antiinflammatorische Wirkung.

Darüber hinaus umfasst die Erfindung die Kombination der erfindungsgemäßen Verbindungen mit organischen Nitraten und NO-Donatoren.

Organische Nitrate und NO-Donatoren im Rahmen der Erfindung sind im Allgemeinen Substanzen, die NO bzw. NO-Voläufer freisetzen. Bevorzugt sind Natriumnitroprussid, Nitroglycerin, Isosorbiddinitrat, Isosorbidmononitrat, Molsidomin und SIN-1.

Außerdem umfasst die Erfindung die Kombination mit Verbindungen, die den Abbau von cyclischem Guanosinmonophosphat (cGMP) inhibieren. Dies sind insbesondere Inhibitoren der Phosphodiesterasen 1, 2 und 5; Nomenklatur nach Beavo und Reifsnyder (1990) TiPS 11 S. 150 bis 155. Durch diese Inhibitoren wird die Wirkung der erfindungsgemäßen Verbindungen potenziert und der gewünschte pharmakologische Effekt gesteigert.

Die *in vitro*-Wirkung der erfindungsgemäßen Verbindungen kann in folgenden Assays gezeigt werden:

### Erhöhung von cGMP in primären Cortexneuronen

Rattenembryonen (Embryonal-Tag 17-19) werden dekapitiert, das Großhirn präpariert und 30 min bei 37°C mit 5 mL Papainlösung und 250 µL DNAse (Papain-Kit von Cell-System) inkubiert, mittels Pasteurpipette homogenisiert und 5 min bei 1200 U/min zentrifugiert. Der Überstand wird abgenommen, das Zellpellet resuspendiert (in 2.7 mL EBSS [Earl's balanced salt solution], 300 µL Ovomucoid/Albumin (Konz.) Lösung, 150 µL DNAse; Papain-Kit von Cell-System), über 5 mL Ovomucoid/Albumin Lösung geschichtet und 6 min bei 700 U/min zentrifugiert. Der Überstand wird abgenommen, die Zellen werden im Kultivierungsmedium (Neurobasalmedium vom Gibco, B27 Supplement 50fach 1 mL/100 mL, 2 mM L-Glutamin) resuspendiert, gezählt (ca. 150'000 Zellen/well) und auf mit Poly-D-Lysin beschichteten 96-well Platten (Costar) mit 200µL/well ausplattiert. Nach 6-7 Tagen bei 37°C (5% CO₂) werden die Neuronen vom Kulturmedium befreit und einmal mit Testpuffer (154 mM NaCl, 5.6 mM KCl, 2.3 mM CaCl₂·2H₂O, 1 mM MgCl₂, 5.6 mM Glucose, 8.6 mM HEPES (4-(2-Hydroxyethyl)-piperazin-1-ethansulfonsäure), pH=7.4) gewaschen. Es werden 100µL/well Testsubstanz in Testpuffer gelöst und danach 100µL/well IBMX (3-Isobutyl-1-methylxanthin; gelöst in 50 mM Ethanol, verdünnt mit Testpuffer auf 100 µM Endkonzentration) zugegeben. Nach 20 min Inkubation bei 37°C wird der Testpuffer durch 200 µL/well Lysispuffer (cGMP EIA RPN 226 von Amersham Pharmacia Biotech) ersetzt, und der cGMP Gehalt der Lysate mittels EIA-Testkit bestimmt.

Die in Tabelle 1 angegebenen Konzentrationen führen zu einer statistisch signifikanten Erhöhung an cGMP (Dreifachbestimmung; mehr als 2-fache Erhöhung gegenüber der Kontrolle)

**Tabelle 1:**

| Beispiel | µM |
|---|---|
| 3 | 0,90 |
| 6 | 0,27 |
| 10 | 1,2 |
| 11 | 0,30 |
| 13 | 0,27 |
| 19 | 0,27 |
| 20 | 0,27 |
| 27 | 0,90 |
| 36 | 0,27 |
| 38 | 0,27 |

### Gefäßrelaxierende Wirkung in vitro

Kaninchen werden durch Nackenschlag betäubt und entblutet. Die Aorta wird entnommen, von anhaftendem Gewebe befreit, in 1,5 mm breite Ringe geteilt und einzeln unter einer Vorspannung in 5 ml-Organbäder mit 37°C warmer, carbogenbegaster Krebs-Henseleit-Lösung folgender Zusammensetzung (mM) gebracht: NaCl: 119; KCl: 4,8; CaCl₂ x 2 H₂O: 1; MgSO₄ x 7 H₂O; 1,4; KH₂PO₄: 1,2; NaHCO₃:25; Glucose: 10. Die Kontraktionskraft wird mit Statham UC2-Zellen erfasst, verstärkt und über A/D-Wandler (DAS-1802 HC, Keithley Instruments München) digitalisiert sowie parallel auf Linienschreiber registriert. Zur Erzeugung einer Kontraktion wird Phenylephrin dem Bad kumulativ in ansteigender Konzentration zugesetzt. Nach mehreren Kontrollzyklen wird die zu untersuchende Substanz (gelöst in 5 µl DMSO) in jedem weiteren Durchgang in jeweils steigender Dosierung untersucht und die Höhe der Kontraktion mit der Höhe der im letzten Kontrollzyklus erreichten Kontraktion (= Kontrollwert) verglichen. Daraus wird die Konzentration errechnet, die erforderlich ist, um die Höhe des Kontrollwertes um 50 % zu reduzieren (IC₅₀).

### Bestimmung der Leberclearance in vitro

Ratten werden anästhesiert, heparinisiert, und die Leber in situ über die Pfortader perfundiert. Ex-vivo werden dann aus der Leber mittels Collagenase-Lösung die primären Ratten-Hepatozyten gewonnen. Es wurden 2·10⁶ Hepatozyten pro ml mit jeweils der gleichen Konzentration der zu untersuchenden Verbindung bei 37°C inkubiert. Die Abnahme des zu untersuchenden Substrates über die Zeit wurde bioanalytisch (HPLC/UV, HPLC/Fluoreszenz oder LC/MSMS) an jeweils 5 Zeitpunkten im Zeitraum von 0-15 min nach Inkubationsstart bestimmt. Daraus wurde über Zellzahl und Lebergewicht die Clearance errechnet.

### Bestimmung der Plasmaclearance in vivo

Die zu untersuchende Substanz wird Ratten über die Schwanzvene intravenös als Lösung appliziert. Zu festgelegten Zeitpunkten wird den Ratten Blut entnommen, dieses wird heparinisiert und durch herkömmliche Maßnahmen Plasma daraus gewonnen. Die Substanz wird im Plasma bioanalytisch quantifiziert. Aus den so ermittelten Plasmakonzentrations-Zeit-Verläufen werden über herkömmliche hierfür verwendete nicht-kompartimentelle Methoden die pharmakokinetischen Parameter errechnet.

Die Eignung der erfindungsgemäßen Verbindungen zur Behandlung von Störungen der Wahrnehmung, Konzentrationsleistung, Lernleistung und/oder Gedächtnisleistung.kann z. B. in folgendem Tiermodell gezeigt werden:

### Bestimmung der Lern-und Gedächnisleistung im Social Recognition Test

Adulte Wistar Ratten (Winkelmann, Borchen; 4-5 Monate) und 4-5 Wochen alte Jungtiere werden während einer Woche an ihre neue Umgebung gewöhnt, wobei jeweils 3 Tiere pro Käfig (Typ IV Makrolon) in 12 h Tag-Nacht Rhythmus (Licht an um 06:00) mit Wasser und Nahrungsmittel *ad libitum* gehalten werden. Getestet werden in der Regel 4 Gruppen à 10 Tiere (1 Vehikelkontrollgruppe, 3 substanzbehandelte Gruppen). Zunächst werden in einem Habituierungslauf alle Tiere wie in Trial 1, aber ohne Substanz oder Vehikel Applikation gehandhabt. Die Testsubstanzen werden direkt nach Trial 1 appliziert. Das soziale Gedächnis wird in Trial 2 nach 24 h gemessen.

**Trial 1:** 30 min vor Testung werden die adulten Ratten einzeln in Käfigen (Typ IV Makrolon) gehalten. 4 min vor Testung wird ein Kasten, bestehend aus zwei Aluminiumseitenwänden, einer Aluminiumrückwand sowie einer Plexiglasfront (63x41x40 cm) über den Käfig gestülpt und der Deckel des Käfigs entfernt. Ein Jungtier wird zur adulten Ratten in den Käfig gegeben, und die soziale Interaktion (z.B. Schnuppern) während 2 min mittels Stopuhr zeitlich erfasst. Danach werden die Tiere zurück in ihren Käfig gebracht.

**Trial 2:** Der Versuch wird nach 24 h analog zu Trial 1 mit den selben Tieren wiederholt. Die Differenz zwischen der sozialen Interaktionszeit bei Trial 1 und Trial 2 wird als Mass für das soziale Gedächnis genommen.

Die erfindungsgemäßen Verbindungen eignen sich zur Verwendung als Arzneimittel für Menschen und Tiere.

Zur vorliegenden Erfindung gehören auch pharmazeutische Zubereitungen, die neben inerten, nicht-toxischen, pharmazeutisch geeigneten Hilfs- und Trägerstoffen eine oder mehrere erfindungsgemäße Verbindungen enthalten, oder die aus einem oder mehreren erfindungsgemäßen Verbindungen bestehen, sowie Verfahren zur Herstellung dieser Zubereitungen.

Die erfindungsgemäßen Verbindungen sollen in diesen Zubereitungen in einer Konzentration von 0,1 bis 99,5 Gew.-%, bevorzugt von 0,5 bis 95 Gew.-% der Gesamtmischung vorhanden sein.

Neben den erfindungsgemäßen Verbindungen können die pharmazeutischen Zubereitungen auch andere pharmazeutische Wirkstoffe enthalten.

Die oben aufgeführten pharmazeutischen Zubereitungen können in üblicher Weise nach bekannten Methoden hergestellt werden, beispielsweise mit dem oder den Hilfs- oder Trägerstoffen.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenen Dosierungsspielraum zu erreichen.

Die Formulierungen können beispielsweise durch Verdünnen der Wirkstoffe mit Lösungsmitteln und/oder Trägerstoffen, gegebenenfalls unter Verwendung von Emulgiermitteln und/oder Dispergiermitteln hergestellt werden, wobei z.B. im Fall der Benutzung von Wasser als Verdünnungsmittel gegebenenfalls organische Lösungsmittel als Hilfslösungsmittel verwendet werden können.

Die Applikation kann in üblicher Weise, vorzugsweise oral, transdermal oder parenteral, insbesondere perlingual oder intravenös erfolgen. Sie kann aber auch durch Inhalation über Mund oder Nase, beispielsweise mit Hilfe eines Sprays erfolgen, oder topisch über die Haut.

Im Allgemeinen hat es sich als vorteilhaft erwiesen, Mengen von etwa 0,001 bis 10 mg/kg, bei oraler Anwendung vorzugsweise etwa 0,005 bis 3 mg/kg Körpergewicht zur Erzielung wirksamer Ergebnisse zu verabreichen.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

### Abkürzungen:

| | |
|---|---|
| ACN | Acetonitril |
| CI | chemische Ionisation (bei MS) |
| DCM | Dichlormethan |
| DME | 1,2-Dimethoxyethan |
| DMF | *N,N*-Dimethylformamid |
| DMSO | Dimethylsulfoxid |
| d.Th. | der Theorie (bei Ausbeute) |
| ESI | Eleh-trospray-Ionisation (bei MS) |
| GC | Gaschromatographie |
| HPLC | Hochdruck-, Hochleistungsflüssigkeitschromatographie |
| LC-MS | Flüssigkeitschromatographie-gekoppelte Massenspektroskopie |
| MS | Massenspektroskopie |
| NMR | Kernresonanzspektroskopie |
| rac-BINAP | rac-2,2-Bis(diphenylphosphino)-1,1-dinaphthyl |
| R_{f} | Retentionsindex (bei DC) |
| RT | Raumtemperatur, 20°C |
| Rₜ | Retentionszeit (bei HPLC) |
| Smp. | Schmelzpunkt |
| TFA | Trifluoressigsäure |
| THF | Tetrahydrofuran |

### Analytische Methoden:

### HPLC

Instrument: HP 1100 mit DAD-Detektion; Säule: Kromasil RP-18, 60 mm x 2 mm, 3.5 µm; Eluent: A = 5 ml Perchlorsäure/l H₂O, B = ACN; Gradient: 0 min 2% B, 0.5 min 2% B, 4.5 min 90% B, 6.5 min 90% B; Fluss: 0.75 ml/min; Temp.: 30°C; UV-Detektion: 210 nm.

### Präparative HPLC

Säule: YMC GEL ODS-AQS-11 µm, 250 mm x 30 mm; Eluent: A = H₂O, B = ACN; Gradient: 0 min 10% B, 10 min 10% B, 35 min 100% B, 45 min 100% B; Fluss: 33 ml/min; Temp.: ca. 22°C; UV-Detektion: 254 nm.

### LC/MS

### Methode A:

Instrument: Finnigan MAT 900S, TSP: P4000, AS3000, UV3000HR; Säule: Symmetry C 18, 150 mm x 2.1 mm, 5.0 µm; Eluent C: Wasser, Eluent B: Wasser + 0.3 g 35%-ige Salzsäure, Eluent A: ACN; Gradient: 0 min 2% A → 2.5 min 95% A → 5 min 95% A; Ofen: 70°C; Fluss: 1.2 ml/min; UV-Detektion: 210 nm.

### Methode B:

Instrument: Finnigan MAT 900S, TSP: P4000, AS3000, UV3000HR; Säule: Symmetry C 18, 150 mm x 2.1 mm, 5.0 µm; Eluent A: Acetonitril, Eluent B: Wasser + 0.6 g 30%-ige Salzsäure; Gradient: 0 min 10% A → 4 min 90% A → 9 min 90% A; Ofen: 50°C; Fluss: 0.6 ml/min; UV-Detektion: 210 nm.

### Methode C:

Instrument: Micromass Quattro LCZ, HP1100; Säule: Symmetry C 18, 50 mm x 2.1 mm, 3.5 µm; Eluent A: Acetonitril + 0.1% Ameisensäure, Eluent B: Wasser + 0.1% Ameisensäure; Gradient: 0 min 10% A → 4 min 90% A → 6 min 90% A; Ofen: 40°C; Fluss: 0.5 ml/min; UV-Detektion: 208-400 nm.

### Methode D:

Instrument: Micromass Platform LCZ, HP1100; Säule: Symmetry C 18, 50 mm x 2.1 mm, 3.5 µm; Eluent A: Acetonitril + 0.1% Ameisensäure, Eluent B: Wasser + 0.1 % Ameisensäure; Gradient: 0 min 10% A → 4 min 90% A → 6 min 90% A; Ofen: 40°C; Fluss: 0.5 ml/min; UV-Detektion: 208-400 nm.

### Methode E:

Instrument: Finnigan MAT 900S, TSP: P4000, AS3000, UV3000HR; Säule: Symmetry C 18, 150 mm x 2.1 mm, 5.0 µm; Eluent A: Acetonitril, Eluent B: Wasser + 0.3 g 30%-ige Salzsäure; Gradient: 0 min 10% A → 3 min 90% A → 6 min 90% A; Ofen: 50°C; Fluss: 0.9 ml/min; UV-Detektion: 210 nm.

### GC/MS

| | |
|---|---|
| Trägergas: | Helium |
| Fluß: | 1.5 ml/min |
| Anfangstemperatur: | 60°C |
| Temperaturgradient: | 14°C/min bis 300°C, dann 1 min konst. 300°C |
| Säule: | HP-5 30 m x 320 µm x 0.25 µm (Filmdicke) |
| Anfangszeit: | 2 min |
| Frontinjektor-Temp.: | 250°C |

### Ausgangsverbindungen:

### Beispiel I

### Schritt 1

### 5-Amino-1-(2-fluorbenzyl)-pyrazol-3-carbonsäureethylester

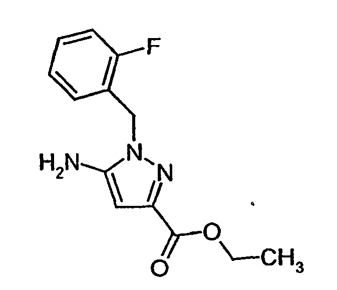

100.00 g (0.613 mol) Natriumsalz des Cyanobrenztraubensäureethylesters [Darstellung analog Borsche und Manteuffel, Liebigs Ann. 1934, *512,* 97] werden unter gutem Rühren unter Argon in 2.5 1 Dioxan bei Raumtemperatur mit 111.75 g (75 ml, 0.98 mol) Trifluoressigsäure versetzt und 10 min gerührt, wobei ein großer Teil des Eduktes in Lösung geht. Dann gibt man 85.93 g (0.613 mol) 2-Fluorbenzylhydrazin hinzu und kocht über Nacht. Nach dem Abkühlen werden die ausgefallenen Kristalle des Natriumtrifluoracetats abgesaugt, mit Dioxan gewaschen und die verbleibende Lösung roh weiter umgesetzt.

### Schritt 2

### 1-(2-Fluorbenzyl)-1H-pyrazolo[3,4-bjpyiidin-3-carbonsäureethylester

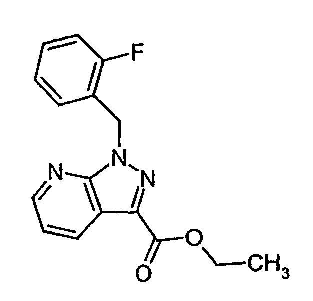

Die in Schritt 1 erhaltene Lösung wird mit 61.25 ml (60.77 g, 0.613 mol) Dimethylaminoacrolein und 56.28 ml (83.88 g, 0.736 mol) Trifluoressigsäure versetzt und unter Argon 3 Tage lang gekocht. Anschließend wird das Lösungsmittel im Vakuum entfernt, der Rückstand in 2 1 Wasser gegeben und dreimal mit je 1 1 Ethylacetat extrahiert. Die vereinigten organischen Phasen werden mit Magnesiumsulfat getrocknet und im Vakuum eingeengt. Man chromatographiert auf 2.5 kg Kieselgel und eluiert mit einem Toluol/Toluol-Ethylacetat (4:1)-Gradienten.
Ausbeute: 91.6 g (49.9% d.Th. über zwei Stufen)
Smp.: 85°C
R_{f} (Kieselgel, Toluol / Essigsäureethylester 1:1): 0.83

### Schritt 3

### 1-(2-Fluorbenzyl)-1H-pyrazolo[3,4-b]pyridin-3-carboxamid

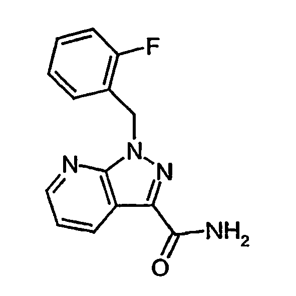

10.18 g (34 mmol) des in Schritt 2 erhaltenen Esters werden in 150 ml mit Ammoniak bei 0-10°C gesättigtem Methanol vorgelegt. Man rührt zwei Tage bei Raumtemperatur und engt anschließend im Vakuum ein.
R_{f} (Kieselgel, Toluol / Essigsäureethylester 1:1): 0.33

### Schritt 4

### 3-Cyano-1-(2-fluorbenzyl)-1H-pyrazolo[3,4-b]pyridin

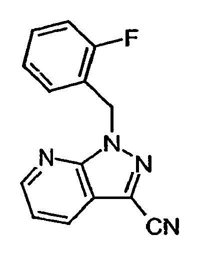

36.10 g (133 mmol) 1-(2-Fluorbenzyl)-1H-pyrazolo[3,4-b]pyridin-3-carboxamid aus Schritt 3 werden in 330 ml THF gelöst und mit 27.00 g (341 mmol) Pyridin versetzt. Anschließend gibt man innerhalb von 10 min 47.76 ml (71.66 g, 341 mmol) Trifluoressigsäureanhydrid hinzu, wobei die Temperatur bis auf 40°C ansteigt. Man rührt über Nacht bei Raumtemperatur. Anschließend wird der Ansatz in 1 1 Wasser gegeben und dreimal mit je 0.5 1 Ethylacetat extrahiert. Die organische Phase wird mit gesättigter wässriger Natriumhydrogencarbonat-Lösung und mit 1 N Salzsäure gewaschen, mit Magnesiumsulfat getrocknet und im Vakuum eingeengt.
Ausbeute: 33.7 g (100% d.Th.)
Smp.: 81°C
R_{f} (Kieselgel, Toluol / Essigsäureethylester 1:1): 0.74

### Schritt 5

### 1-(2-Fluorbenzyl)-1H-pyrazolo[3,4-b]pyridin-3-carboximidamid

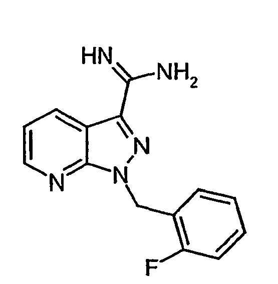

108.00 g (0.43 mol) 1-(2-Fluorbenzyl)-1H-pyrazolo[3,4-b]pyridin-3-carbonitril (Beispiel I, Schritt 4) werden in einem Liter Methanol gelöst und zu einer Lösung aus 94.73 g (1.67 mol; Reinheit: 95%) Natriummethylat in 3 1 Methanol getropft. Man rührt 2 Stunden bei RT nach, gibt dann 28.83 g (0.54 mol) Ammoniumchlorid zu und versetzt anschließend tropfenweise mit 100.03 g (1.67 mol) Eisessig. Die Lösung wird über Nacht unter Rückfluss gerührt. Das Lösungsmittel wird im Vakuum entfernt, der Rückstand zweimal in Aceton suspendiert und der unlösliche Feststoff abgesaugt. Dieser wird in 1.5 1 Ethylacetat gelöst und mit 590 ml einer wässrigen 20%-igen Natriumcarbonat-Lösung versetzt. Man rührt 20 Minuten nach und verdünnt dann mit 200 ml 1 N Natriumhydroxid-Lösung. Die organische Phase wird mit gesättigter wässriger Natriumchlorid-Lösung gewaschen, über Magnesiumsulfat getrocknet und filtriert. Das Lösungsmittel wird im Vakuum entfernt. Man erhält 99.10 g (86% d.Th.) des Produktes.
LC/MS (Methode B): Rₜ = 2.25 min.
MS (ESIpos): m/z = 270 (M+H)⁺
¹H-NMR (300 MHz, DMSO-d₆): δ = 5.79 (s, 2H), 6.54 (br s, 3H), 7.09-7.18 (m, 2H), 7.23 (t, 1H), 7.31-7.41 (m, 2H), 8.62 (d, 1H), 8.69 (d, 1H).

### Schritt 6

### 3-(5-Brom-2-pyrimidinyl)-1-(2-fluorbenzyl)-1H-pyrazolo[3,4-b]pyridin

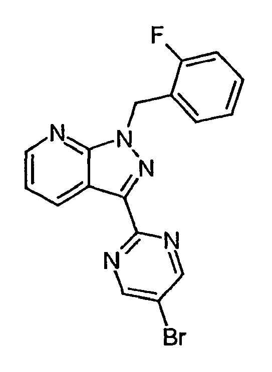

10.09 g (66.84 mmol) 2-Brommalonaldehyd werden zu einer Lösung aus 15.00 g (55.70 mmol) 1-(2-Fluorbenzyl)-1H-pyrazolo[3,4-b]pyridin-3-carboximidamid (Beispiel I, Schritt 5) in 200 ml Eisessig gegeben und 2 Stunden bei 100°C gerührt. Das Lösungsmittel wird im Vakuum entfernt. Der Rückstand wird chromatographisch über Kieselgel aufgereinigt (Eluent: DCM/Methanol 40:1 bis 30:1). Man erhält 9.51 g (44% d.Th.) des Produktes.
HPLC:Rₜ= 4.85 min.
LC/MS (Methode C): Rₜ = 4.57 min.
MS (ESIpos): m/z = 385 (M+H)⁺
¹H-NMR (300 MHz, DMSO-d₆): δ = 5.87 (s, 2H), 7.17 (q, 1H), 7.21-7.30 (m, 2H), 7.37 (q, 1H), 7.45 (dd, 1H), 8.70 (d, 1H), 8.82 (d, 1H), 9.13 (s, 2H).

### Beispiel II

### Schritt 1

### 7-Benzyl-3-phenylsulfonyl-9-oxa-3,7-diazabicyclo[3.3.1 ]nonan

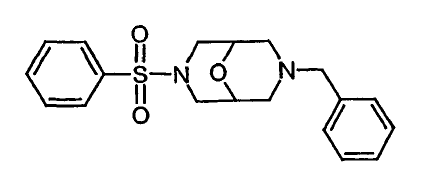

Man erhitzt 104.00 g (0.2 mol) N-Phenylsulfonyl-2,6-bis-iodmethyl-morpholin [Stetter, H.; Meissner, H.-J. Chem. Ber. 96, 2827 (1963)] mit 64.00 g (0.6 mol) Benzylamin in 1 1 Xylol über Nacht unter Rückfluss. Man saugt das Benzylammoniumiodid ab, engt das Filtrat ein und kristallisiert den Rückstand aus Ethanol um.
Man erhält 32 g (44.6% d.Th.) des Produktes.
Smp.: 185-186°C

### Schritt 2

### 7-Benzyl-9-oxa-3,7-diazabicyclo[3.3.1]nonan

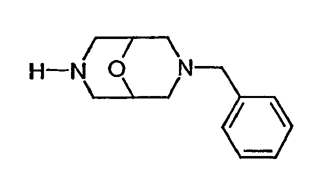

Man legt 10.00 g (0.25 mol) Lithiumaluminiumhydrid in 300 ml absolutem Tetrahydrofuran vor, erhitzt zum Rückfluss und tropft 18.00 g (50 mmol) 7-Benzyl-3-phenylsulfonyl-9-oxa-3,7-diaza-bicyclo[3.3.1]nonan in 150 ml absolutem Tetrahydrofuran gelöst hinzu. Man erhitzt 48 Stunden unter Rückfluss, tropft je 10 ml Wasser, 15%-ige Kalilauge und wieder Wasser hinzu, saugt die anorganischen Salze ab und kocht sie zweimal mit Tetrahydrofuran aus. Die Tetrahydrofuran-Lösungen werden eingeengt und der Rückstand im Hochvakuum destilliert. Man erhält 5.30 g (43.5% d.Th.) des Produktes.
Siedepunkt: 110°C / 0.1 mbar

### Schritt 3

### 7-Benzyl-9-oxa-3,7-diazabicyclo[3.3.1 ]nonan-3-carbonsäure-tert.-butylester

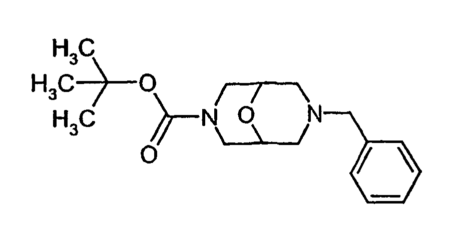

Zu 5.00 g (23 mmol) 7-Benzyl-9-oxa-3,7-diazabicyclo[3.3.1]nonan in 25 ml tert.-Butanol gibt man 1.00 g Natriumhydroxid in 5 ml Wasser und tropft 5.30 g (24 mmol) Pyrokohlensäuredi-tert.-butylester hinzu. Man rührt über Nacht bei Raumtemperatur, versetzt mit 50 ml Wasser, extrahiert dreimal mit Chloroform, trocknet über Magnesiumsulfat, saugt das Trockenmittel ab, engt das Filtrat ein und verrührt den kristallinen Rückstand mit n-Hexan. Man saugt die Kristalle ab und trocknet sie an der Luft. Man erhält 5.80 g des Produktes (73% d.Th.).
Smp.: 100-102°C

### Schritt 4

### 9-Oxa-3,7-diazabicyclo[3.3.1]nonan-3-carbonsäure-tert.-butylester

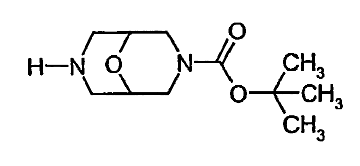

Man löst 5.60 g (17.6 mmol) 7-Benzyl-9-oxa-3,7-diazabicyclo[3.3.1]nonan-3-carbonsäure-tert.-butylester in 100 ml Ethanol, setzt 1.00 g 10%-iges Palladium auf Aktivkohle hinzu und hydriert bei 100°C und 100 bar. Man saugt den Katalysator ab und engt das Filtrat ein, worauf 3.80 g (94.5% d.Th.) reines Produkt auskristallisieren.
Smp.: 93-95°C

### Beispiel III

### Schritt 1

### 1-Benzyl-3-hydroxy-3-(2-hydroxyethylaminomethyl)-pyrrolidin

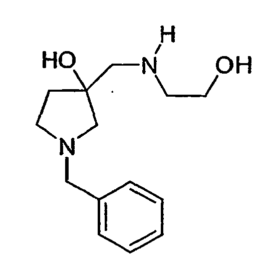

Man tropft 32.7 g (0.17 mol) 5-Benzyl-1-oxa-5-azaspiro[4.2]heptan (US-Patent 4,508,724) zu 31.0 g (0.52 mol) Ethanolamin in 250 ml Wasser und rührt über Nacht bei Raumtemperatur. Man extrahiert mit Diethylether, engt die wässrige Phase ein und destilliert den Rückstand im Hochvakuum. Man erhält 42.1 g (95.9% d.Th.) des Produktes.
Siedepunkt: 180-190°C / 0.1 mbar

### Schritt 2

### 7-Benzyl-1-oxa-4,7-diazaspiro[5.4]decan

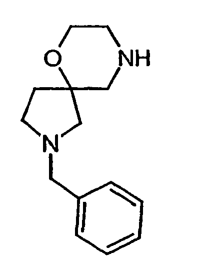

Man löst 85.0 g (340 mmol) 1-Benzyl-3-hydroxy-3-(2-hydroxyethylaminomethyl)-pyrrolidin in einem Gemisch von 280 ml konzentrierter Schwefelsäure und 140 ml Wasser und erhitzt über Nacht auf 180°C. Man stellt mit 45%-iger Natronlauge alkalisch, löst ausgeschiedene Salze mit Wasser und extrahiert fünfmal mit je 200 ml Chloroform. Man trocknet die organischen Phasen über Kaliumcarbonat, trennt das Trockenmittel ab und engt die Lösung ein. Der Rückstand wird im Hochvakuum destilliert. Man erhält 60.0 g (76% d.Th.) des Produktes.
Siedepunkt: 125°C / 0.08 mbar

### Schritt 3

### 7-Benzyl-1-oxa-4,7-diazaspiro[5.4]decan-4-carbonsäure-tert.-butylester

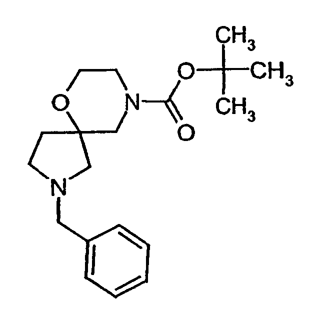

Zu 10.3 g (47 mmol) 7-Benzyl-1-oxa-4,7-diazaspiro[5.4]decan in 30 ml tert.-Butanol gibt man 2.0 g Natriumhydroxid in 25 ml Wasser und tropft 11.0 g (50 mmol) Pyrokohlensäuredi-tert.-butylester hinzu. Man rührt über Nacht bei Raumtemperatur, versetzt mit 50 ml Wasser, extrahiert dreimal mit Chloroform, trocknet über Kaliumcarbonat, saugt das Trockenmittel ab, engt das Filtrat ein und destilliert den Rückstand im Hochvakuum. Man erhält 13.8 g des Produktes (88% d.Th.).
Siedepunkt: 160°C / 0.3 mbar

### Schritt 4

### 1-Oxa-4,7-diazaspiro[5.4]decan-4-carbonsäure-tert.-butylester

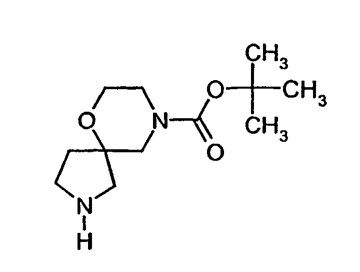

Man löst 13.7 g (41 mmol) 7-Benzyl-1-oxa-4,7-diazaspiro[5.4]decan-4-carbonsäure-tert.-butylester in 300 ml Methanol, setzt 3.0 g 10 %-iges Palladium auf Aktivkohle hinzu und hydriert bei 100°C und 100 bar. Man saugt den Katalysator ab, engt das Filtrat ein und destilliert den Rückstand im Hochvakuum. Man erhält 7.6 g (75 % d.Th.) des Produktes.
Siedepunkt: 113°C / 0.07 mbar

### Beispiel IV

### Schritt 1

### 10-Oxa-4-azatricyclo[5.2.1.0^{2,6}]decan-3,5-dion

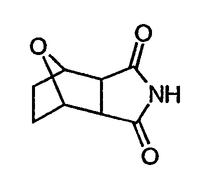

Diese Verbindung ist durch Diels-Alder-Reah-tion von Furan mit Maleinsäureimid zugänglich, zum Beispiel analog Fisera, L., Melnikov, J., Pronayova, N., Ertl, P., Chem. Pap. 1995, 49 (4), 186-191.

### Schritt 2

### 10-Oxa-4-azatricyclo[5.2.1.0^{2,6}]decan

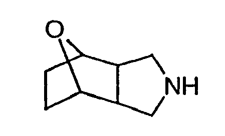

5.00 g (29.91 mmol) 10-Oxa-4-azatricyclo[5.2.1.0^{2,6}]decan-3,5-dion (Beispiel IV, Schritt 1) werden unter Argon in 150 ml absolutem THF suspendiert und per Eisbad gekühlt. Dazu gibt man portionsweise 2.27 g (59.82 mmol) Lithiumaluminiumhydrid und rührt bei 0°C über Nacht. Die Reaktionslösung wird mit gesättigter Natriumchlorid-Lösung und destilliertem Wasser hydrolysiert, die entstandene Suspension filtriert und der Feststoff mit Ethylacetat gewaschen. Die organische Phase des Filtrats wird abgetrennt und die wässrige Phase nochmals mit Ethylacetat extrahiert. Die vereinigten organischen Phasen werden getrocknet, filtriert und das Lösungsmittel im Vakuum entfernt. Der Rückstand wird in Diethylether aufgeschlämmt, filtriert, mit Diethylether gewaschen und getrocknet. Man erhält 730 mg (13% d.Th.) des Produktes.
GC/MS: Rₜ = 6.22 min.
MS (EI): m/z = 139 (M)⁺
¹H-NMR (200 MHz, DMSO-d₆): δ = 1.30-1.60 (m, 4H), 2.06-2.22 (quintett, 2H), 2.26-2.40 (dd, 2H), 2.81-2.98 (dd, 2H), 4.11-4.22 (t, 2H).

### Beispiel V

### Decahydropyrrolo[3,4-b]pyrrolizidin

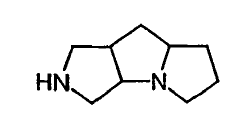

Die Herstellung dieser Verbindung ist beschrieben in: Schenke, Th., Petersen, U., US-Patent 5,071.,999.

### Beispiel VI

### Schritt 1

### 2-Benzyl-2,5-diazabicyclo[2.2.1]heptan

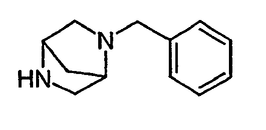

Die Darstellung dieser Verbindung ist beschrieben in: Portoghese, P.S., Mikhail, A.A., J. Org. Chem. 31 (1966), 1059.

### Schritt 2

### tert-Butyl 5-benzyl-2,5-diazabicyclo[2.2.1]heptan-2-carboxylat

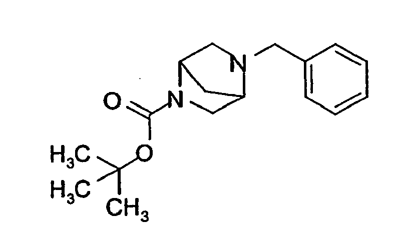

Die Darstellung dieser Verbindung erfolgt analog der Vorschrift des Beispiels III, Schritt 3.

### Schritt 3

### tert-Butyl-2,5-diazabicyclo[2.2.1]heptan-2-carboxylat

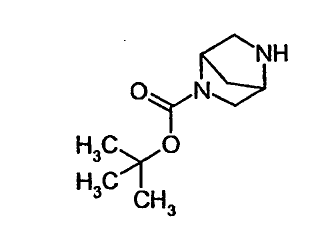

Die Darstellung dieser Verbindung erfolgt analog der Vorschrift des Beispiels III, Schritt 4.

### Schritt 4

### tert-Butyl-5- {2-[1-(2-fluorbenzyl)-1H-pyrazolo[3,4-b]pyridin-3-yl]-5-pyrimidinyl}-2,5-diazabicyclo[2.2.1]heptan-2-carboxylat

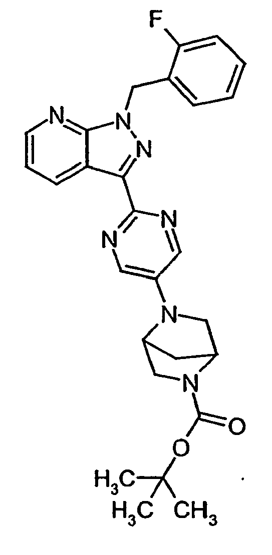

95 mg (0.85 mmol) gut getrocknetes Kalium-tert-butylat werden in einer zuvor ausgeheizten und evakuierten Apparatur unter Argon vorgelegt. 18 mg (0.02 mmol) Tris-(dibenzyliden-aceton)dipalladium, 48 mg (0.08 mmol) rac-BINAP und 300 mg (0.77 mmol) 3-(5-Brom-2-pyrimidinyl)-1-(2-fluorbenzyl)-1H-pyrazolo[3,4-b]pyridin (Beispiel I, Schritt 6) werden nacheinander zugegeben und die Apparatur nochmals evakuiert und mit Argon gespült. Die Reagenzien werden in 40 ml absolutem Toluol suspendiert und dann werden 460 mg (2.32 mmol) tert-Butyl-2,5-diazabicyclo[2.2.1]heptan-2-carboxylat (Beispiel VI, Schritt 3) zu der Reaktionsmischung zugegeben. Diese rührt man bei 60°C über Nacht. Das Lösungsmittel wird im Vakuum entfernt und der Rückstand mittels präparativer HPLC gereinigt. Man erhält 327 mg (84% d.Th.) des Produktes.
LC/MS (Methode C): Rₜ = 4.50 min.
MS (ESIpos): m/z = 502.1 (M+H)⁺
R_{f}= 0.38 (DCM/Methanol 20:1)
¹H-NMR (300 MHz, DMSO-d₆): δ = 1.37 (d, 9H), 1.96 (s, 2H), 3.21 (t, 2H), 3.30-3.45 (m, 1H), 3.64 (br d, 1H), 4.51 (br d, 1H), 4.78 (s, 1H), 5.81 (s, 2H), 7.08-7.53 (m, 5H), 8.38 (s, 2H), 8.63 (d, 1H), 8.82 (d, 1H).

Die in der folgenden Tabelle aufgeführten Beispiele können analog der zuvor beschriebenen Vorschrift des Beispiels VI aus den entsprechenden Ausgangsverbindungen hergestellt werden:

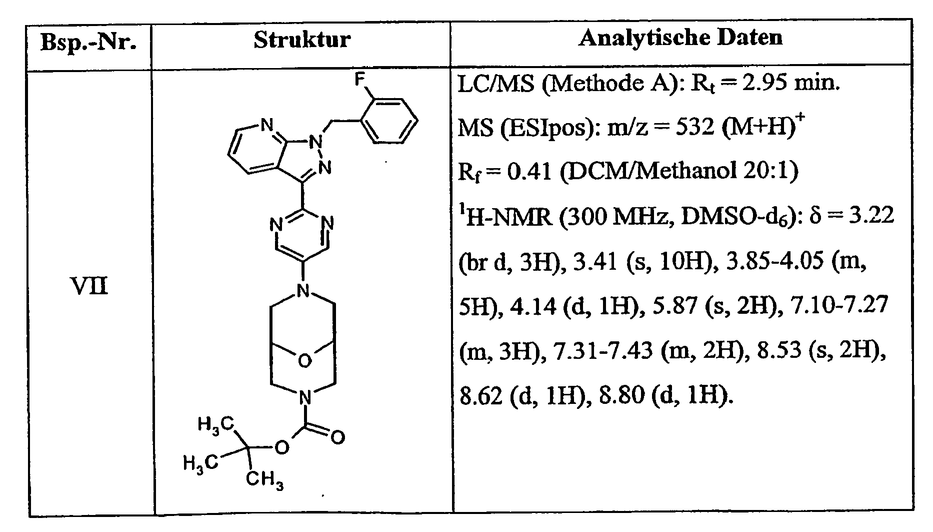

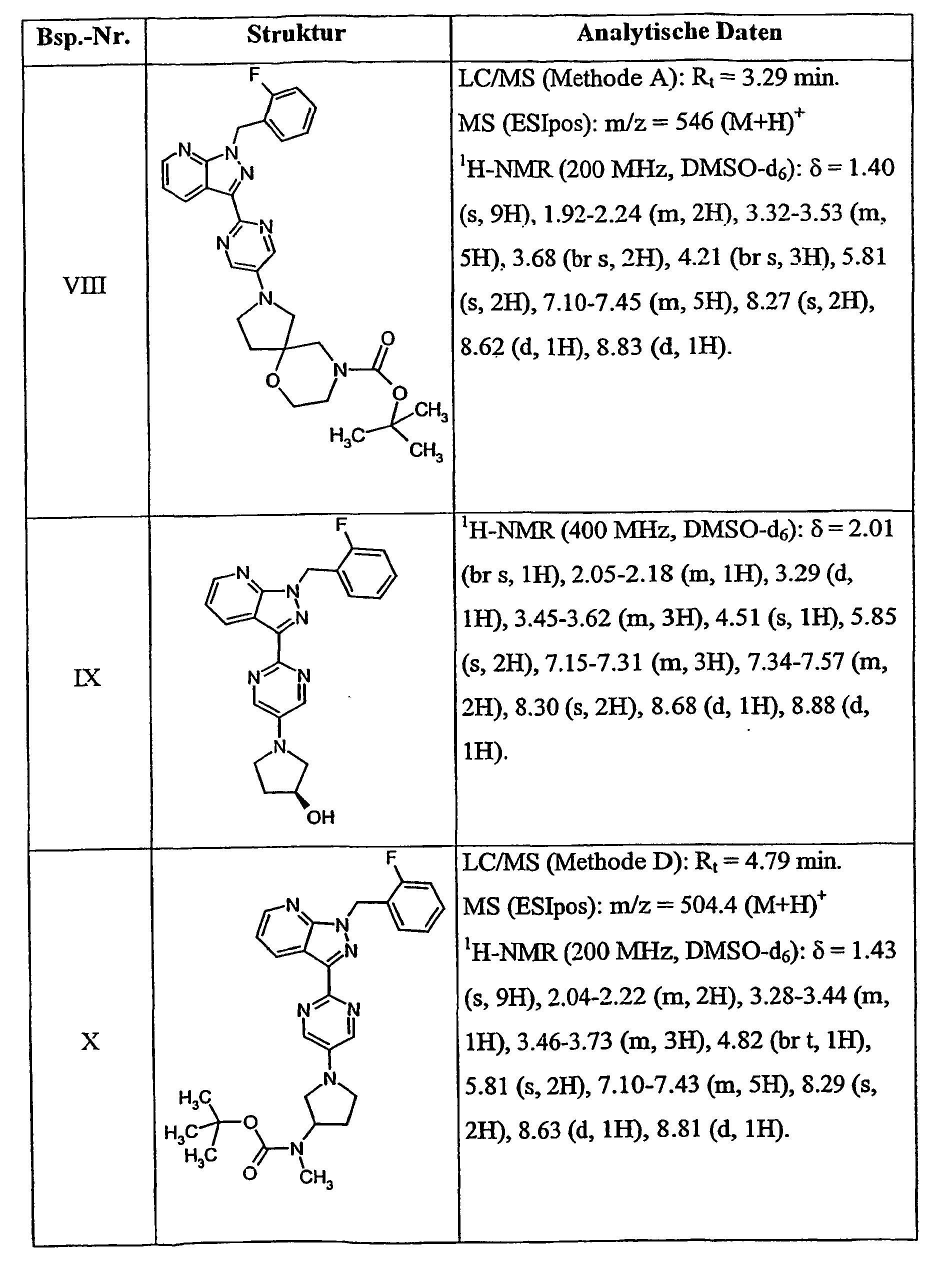

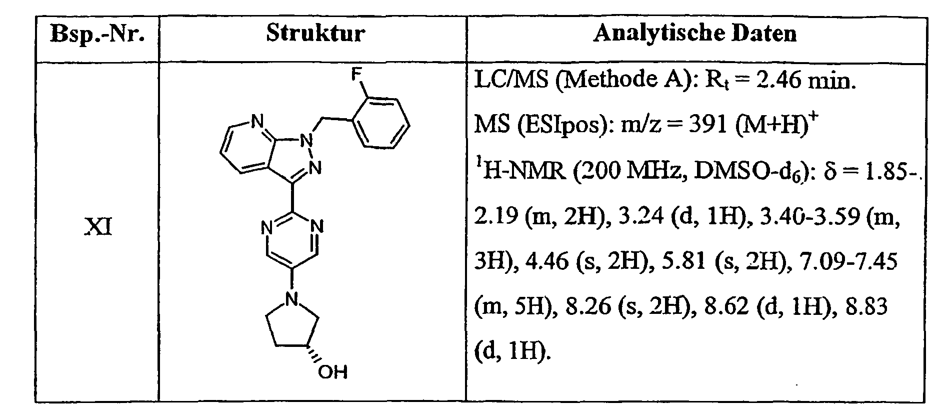

### Beispiel XII

### Schritt 1

### 2,5-Anhydro-3,4-dideoxy-1,6-bis-O-[(4-methylphenyl)sulfonyl]hexitol

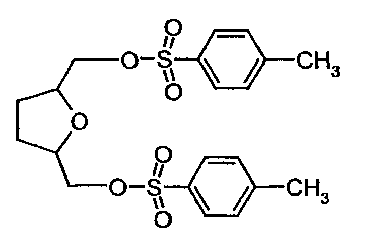

34.0 g (261 mmol) 2,5-Bis(hydroxymethyl)tetahydrofuran werden in 260 ml Dichlormethan gelöst. Hierzu wird eine Lösung von 99.0 g (521 mmol) p-Toluolsulfonsäurechlorid in 52 ml Pyridin und 130 ml Dichlormethan zugetropft. Nach 24-stündigem Rühren bei Raumtemperatur wird der Niederschlag abgesaugt und mit Dichlormethan gewaschen. Das Filtrat und die Waschphasen werden vereinigt, mit verdünnter Salzsäure und anschließend mit gesättigter wässriger Natriumhydrogencarbonat-Lösung gewaschen, über Magnesiumsulfat getrocknet und zur Trockene eingeengt. Das Rohprodukt wird aus Ethanol umkristallisiert.
Ausbeute: 112 g (98% d.Th.)
Smp.: 125°C
MS (CIpos): m/z = 441 (M+H)⁺.

### Schritt 2

### 3-Benzyl-8-oxa-3-azabieyclo[3.2.1]octan

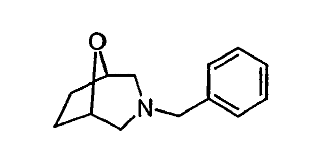

112 g (250 mmol) 2,5-Anhydro-3,4-dideoxy-1,6-bis-O-[(4-methylphenyl)sulfonyl]-hexitol aus Schritt 1 und 90.7 g (840 mmol) Benzylamin werden in 500 ml Toluol 20 Stunden unter Rückfluss erhitzt. Anschließend wird der Niederschlag abgesaugt und mit Toluol gewaschen. Die vereinigten Toluolphasen werden am Rotationsverdampfer eingeengt und im Vakuum destilliert. Nach einem Vorlauf von Benzylamin wird das Produkt erhalten.
Ausbeute: 28.2 g (54% d.Th.)
Siedepunkt: 96-99°C / 8 mbar
MS (CIpos): m/z = 204 (M+H)⁺.

### Schritt 3

### 8-Oxa-3-azabicyclo[3.2.1]octan-Hydrochlorid

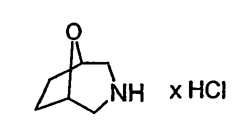

28.20 g (13b mmol) 3-Benzyl-8-oxa-3-azabicyclo[3.2.1]octan aus Schritt 2 werden in 200 ml Ethanol gelöst, mit 5.00 g Palladium auf Aktivkohle (10%-ig) versetzt und bei 100°C mit 100 bar Wasserstoff im Autoklaven hydriert. Der Katalysator wird abgesaugt und die Mutterlauge mit 11.9 ml konzentrierter Salzsäure versetzt und am Rotationsverdampfer eingeengt. Der Rückstand wird mit Aceton versetzt und der entstandene Niederschlag abgesaugt und über Phosphorpentoxid getrocknet.
Ausbeute: 17.0 g (84% d.Th.)
Smp.: 209-221 °C
MS (CIpos): m/z = 114 (M+H)⁺.

### Schritt 4

### 8-Oxa-3-azabicyclo[3.2.1]octan

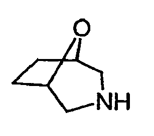

4.15 g (27.7 mmol) 8-Oxa-3-azabicyclo[3.2.1]octan-Hydrochlorid aus Schritt 3 werden in 100 ml Dichlormethan suspendiert und mit einer Lösung von 3.23 g (30.5 mmol) Natriumcarbonat in 30 ml Wasser versetzt. Die Mischung wird 30 Minuten bei Raumtemperatur gerührt. Anschließend wird die organische Phase abgetrennt, mit 30 ml gesättigter wässriger Natriumchlorid-Lösung gewaschen und am Rotationsverdampfer zur Trockene eingeengt. Der Rückstand wird im Vakuum getrocknet.
Ausbeute: 2.46 g (76% d.Th.).

### Schritt 5

### Diethyl 2-(8-oxa-3-azabicyclo[3.2.1]oct-3-yl)malonat

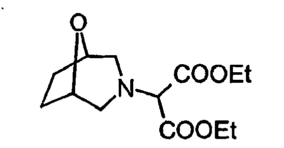

4.72 g (19.8 mmol) 2-Brommalonsäurediethylester werden in 30 ml Acetonitril vorgelegt. Dann werden 3.82 g (27.7 mmol) Kaliumcarbonat und 2.46 g (21.7 mmol) 8-Oxa-3-azabicyclo[3.2.1]octan aus Schritt 4 zugegeben. Die Suspension wird bei 50°C über Nacht gerührt. Anschließend wird abgesaugt und das Filtrat am Rotationsverdampfer zur Trockene eingeengt. Der Rückstand wird ohne weitere Reinigung in der nächsten Stufe eingesetzt.
Ausbeute: 5.09 g (95% d.Th.).

### Schritt 6

### 2-[1-(2-Fluorbenzyl)-1H-pyrazolo[3,4-b]pyridin-3-yl]-5-(8-oxa-3-azabicyclo [3.2.1]-oct-3-yl)-4,6-pyrimidindiol

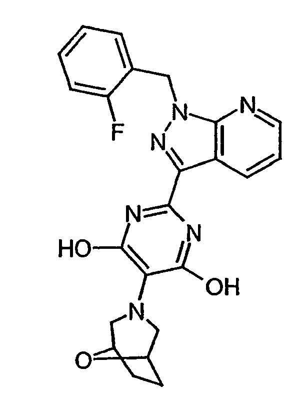

0.50 g (1.11 mmol) 1-(2-Fluorbenzyl)-1H-pyrazolo[3,4-b]pyridin-3-carboximidamid (Beispiel I, Schritt 5) werden in 40 ml Toluol suspendiert. Dann werden 1.40 g (5.16 mmol) Diethyl-2-(8-oxa-3-azabicyclo[3.2.1]oct-3-yl)malonat aus Schritt 5 zugegeben. Die Mischung wird über Nacht bei 140°C gerührt. Anschließend wird der Feststoff abgesaugt, mit Diethylether gewaschen und im Vakuum getrocknet.
Ausbeute: 515 mg (24% d.Th.)
MS (ESIpos): m/z = 449 (M+H)⁺.

### Schritt 7

### 3-[4,6-Dichlor-5-(8-oxa-3-azabicyclo[3.2.1]oct-3-yl)-2-pyrimidinyl]-1-(2-fluorbenzyl)-1H-pyrazolo[3,4-b]pyridin

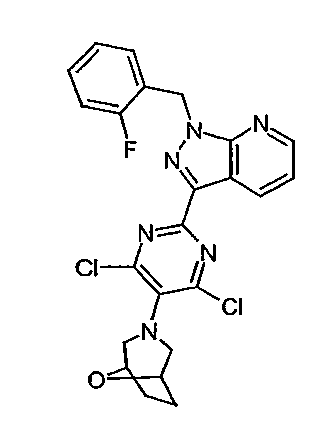

1.33 g (4.69 mmol) 2-[1-(2-Fluorbenzyl)-1H-pyrazolo[3,4-b]pyridin-3-yl]-5-(8-oxa-3-azabicyclo[3.2.1]oct-3-yl)-4,6-pyrimidindiol aus Schritt 6 werden in 5 ml (53.6 mmol) Phosphoroxychlorid gelöst. Die Mischung wird mit 3 Tropfen N,N-Dimethylformamid versetzt und 3 Stunden unter Rückfluss erhitzt. Nach Abkühlen auf Raumtemperatur und Einengen am Rotationsverdampfer wird der Rückstand im Hochvakuum getrocknet Das Rohprodukt wird durch präparative HPLC gereinigt.
Ausbeute: 252 mg (46% d.Th.)
LC/MS (Methode E): Rₜ = 3.58 min
MS (ESIpos): m/z = 485 (M+H)⁺
¹H-NMR (300 MHz, DMSO-d₆): δ = 1.80-1.92 (m, 2H), 2.11-2.20 (m, 2H), 2.66 (d, 2H), 3.54 (dd, 2H), 4.35 (s, 2H), 5.88 (s, 2H), 7.10-7.28 (m, 3H), 7.31-7.41 (m, 1H), 7.55 (dd,1H), 8.70-8.80 (m, 2H).

### Beispiel XIII

### 1-Methyloctahydropyrrolo[3,4-d][1,3]oxazin

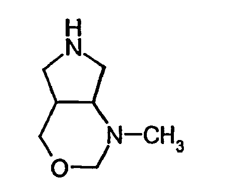

Die Herstellung dieser Verbindung ist beschrieben in: Petersen, U. et al., EP 350 733.

### Beispiel XIV

### tert-Butyl-(3R)-3-hydroxy-1-piperidincarboxylat

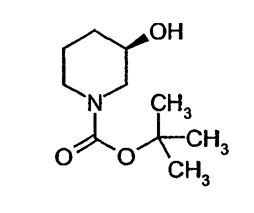

26.80 g (0.20 mol) (3R)-3-Piperidinol-Hydrochlorid werden in 150 ml THF vorgelegt und unter Eisbad-Kühlung mit 65.04 g (0.64 mol) Triethylamin versetzt. Man rührt für 20 Minuten bei gleicher Temperatur nach und trägt dann 70 ml Di(tert-butyl)dicarbonat in die Lösung ein und rührt bei RT über Nacht. Die Reaktionslösung wird mit 1 N Salzsäure extrahiert. Die organische Phase wird mit gesättigter wässriger Natriumchlorid-Lösung gewaschen, getrocknet, filtriert und das Lösungsmittel im Vakuum entfernt. Man erhält 36.00 g (90% d.Th.) des Produktes.
GC/MS: Pₜ = 12.43 min.
MS (EI): m/z = 201 (M)⁺
¹H-NNR (400 MHz, DMSO-d₆): δ = 1.21-1.32 (m, 2H), 1.34-1.42 (m, 9H), 1.48 (s, 1H), 1.58-1.65 (m, 1H), 1.77-1.88 (m, 1H), 2.78 (br t, 1H), 3.33-3.41 (m, 1H), 3.58-3.63 (dt, 1H), 3.75 (br d,1H), 4.82 (s, 1H).

### Beispiel XV

### tert-Butyl-(3R)-3-hydroxy-1-pyrrolidincarboxylat

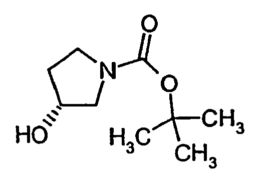

Diese Verbindung wird analog der Vorschrift des Beispiels XIV aus 3-(R)-Hydroxypyrrolidin erhalten.

### Beispiel XVI

### Schritt 1

### tert-Butyl-(3S,4S)-3,4-dihydroxy-1-pyrrolidincarboxylat

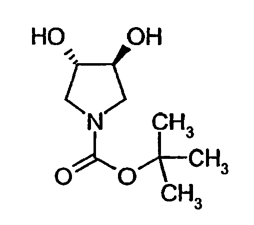

Die Darstellung dieser Verbindung erfolgt nach: Nagel, U., Angew. Chem. 96 (1954), 425.

### Schritt 2

### tert-Butyl-(3S,4S)-3,4-dimethoxy-1-pyrrolidincarboxylat

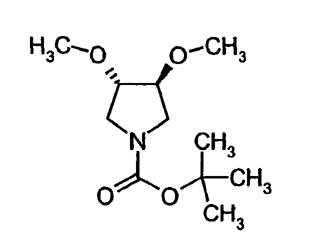

2.60 g (12.73 mmol) tert-Butyl-(3S,4S)-3,4-dihydroxy-1-pyrrolidincarboxylat (Beispiel XVI, Schritt 1) werden unter Argon in 60 ml absolutem DMF gelöst und unter Eisbad-Kühlung portionsweise mit 0.74 g (29.42 mmol; 95%-ig) Natriumhydrid versetzt. Man lässt 10 Minuten bei 0°C nachrühren und tropft dann bei gleicher Temperatur 4.00 g (28.14 mmol) Iodmethan zu. Die Reaktionsmischung wird 2 Stunden bei RT nachgerührt und dann durch Zugabe von 20 ml gesättigter wässriger Natriumchlorid-Lösung und 80 ml destilliertem Wasser hydrolysiert. Die wässrige Phase wird mit Ethylacetat extrahiert. Die vereinigten organischen Phasen werden mit gesättigter wässriger Natriumchlorid-Lösung gewaschen, getrocknet, filtriert und das Lösungsmittel unter Vakuum entfernt. Der Rückstand wird chromatographisch über Kieselgel (Eluent: DCM/Methanol 100:1) aufgereinigt. Man erhält 2.39 (76% d.Th.) des Produktes.
LC/MS (Methode D): Rₜ = 3.45 min.
MS (ES1pos): m/z = 255 (M+H)⁺
¹H-NMR (300 MHz, DMSO-d₆): δ = 1.39 (s, 10H), 2.73 (s, 1H), 2.89 (s, 1H), 3.28 (s, 7H), 3.80 (br s, 2H).

### Schritt 3

### (3S,4S)-3,4-Dimethoxypyrrolidin

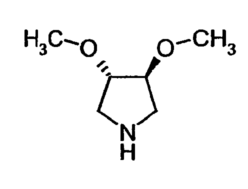

2.30 g (9.35 mmol; 94%-ig) tert-Butyl-(3S,4S)-3,4-dimethoxy-1-pyrrolidincarboxylat (Beispiel XVI, Schritt 2) werden in 50 ml Salzsäure (4 M in Dioxan) aufgenommen und über Nacht bei RT gerührt. Das Lösungsmittel wird im Vakuum entfernt und der Rückstand in DCM suspendiert, mit 1 N Natriumhydroxid-Lösung versetzt und 10 Minuten bei RT gerührt. Die Phasen werden getrennt und die wässrige Phase mehrmals mit DCM extrahiert. Die vereinigten organischen Phasen werden mit gesättigter wässriger Natriumchlorid-Lösung extrahiert, getrocknet, filtriert und das Lösungsmittel im Vakuum entfernt. Man erhält 872 mg (63% d.Th.) des Produktes.
GC/MS: Rₜ = 4.76 min.
MS (CIpos): m/z = 132 (M+H)⁺
¹H-NMR (400 MHz, DMSO-d₆): δ = 2.63 (dd, 4H), 2.89 (dd, 4H), 3.63 (q, 4H), 7.95 (s, 1H).

Die in der folgenden Tabelle aufgeführten Beispiele können analog der zuvor beschriebenen Vorschrift des Beispiels XVI aus den entsprechenden Ausgangsverbindungen hergestellt werden:

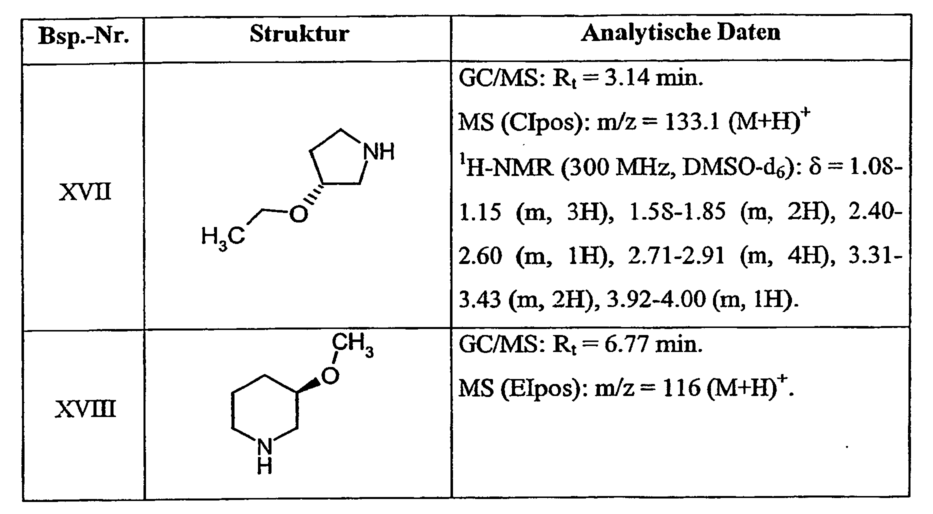

### Beispiel XIX

### Scitritt 1

### (3R)-3-[(Triethylsilyl)oxy]pyrrolidin

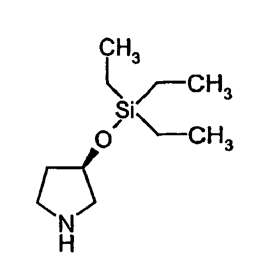

Zu einer eisgekühlten Lösung von 200 mg (2.30 mmol) (3R)-3-Pyrrolidinol in 3 ml absolutem Pyridin unter Argon gibt man 381 mg (2.53 mmol) Triethylchlorsilan. Man rührt die Reaktionsmischung für eine Stunde bei RT, versetzt sie dann mit 6 ml destilliertem Wasser und extrahiert sie mit Diethylether. Das Lösungsmittel der organischen Phase wird unter Vakuum entfernt und der Rückstand gut getrocknet. Man erhält 99 mg (21% d.Th.) des Produktes.
MS (DCI): m/z = 219.2 (M+H)⁺
R_{f}= 0.39 (DCM/Methanol 20:1)
¹H-NMR (200 MHz, DMSO-d₆): δ = 0.58 (q, 6H), 0.92 (t, 9H), 1.81-2.02 (m, 1H), 2.85 (br d, 1H), 3.03-3.18 (m, 4H), 4.48 (br quintett, 1H), 8.52 (br s, 1H).

### Schritt 2

### 1-(2-Fluorbenzyl)-3-(5-{(3R)-3-[(triethylsilyl)oxy]-1-pyrrolidinyl}-2-pyrimidinyl)-1H-pyrazol[3,4-b]pyridin

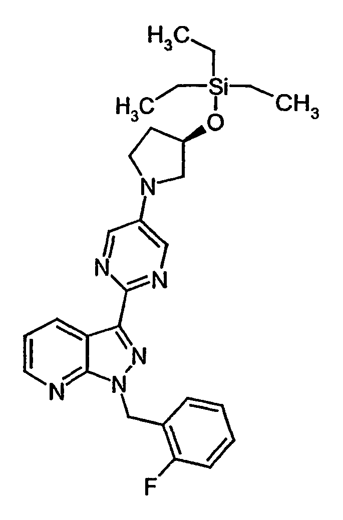

Die Verbindung wird bis auf die folgenden Veränderungen analog der Vorschrift des Beispiels VI, Schritt 4 hergestellt. Die Reaktion wird in absolutem Dioxan mit Natrium-tert-butylat anstatt der entsprechenden Kalium-Verbindung durchgeführt. Ausgehend von 94 mg (0.25 mmol) 3-(5-Brom-2-pyrimidinyl)-1-(2-fluorbenzyl)-1H-pyrazolo[3,4-b]pyridin (Beispiel I, Schritt 6) und 99 mg (0.49 mmol) (3R)-3-[(Triethylsilyl)oxy]pyrrolidin (Beispiel XIX, Schritt 1) erhält man 80 mg (65% d.Th.) des Produktes.
HPLC: Rₜ = 3.98 min.
MS (ESIpos): m/z = 505 (M+H)⁺
R_{f}= 0.32 (DCM/Ethylacetat 2:1)
¹H-NMR (300 MHz, DMSO-d₆): δ = 0.61 (q, 6H), 0.94 (t, 9H), 1.24 (s, 1H), 1.89-2.02 (m, 1H), 2.08-2.22 (m, 1H), 3.47 (t, 2H), 3.59 (dd, 1H), 4.63 (s, 1H), 5.80 (s, 2H), 7.10-7.28 (m, 3H), 7.30-7.41 (m, 2H), 8.27 (s, 2H), 8.62 (d, 1H), 8.82 (d, 1H).

### Schritt 3

### (3R)-1-{2-[1-(2-Fluorbenzyl)-1H-pyrazolo[3,4-b]pyridin-3-yl]-5-pyrimidinyl}-3-pyrrolidinol

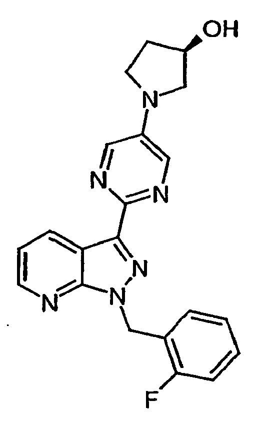

2.64 g (5.23 mol) 1-(2-Fluorbenzyl)-3-(5-{(3R)-3-[(triethylsilyl)oxy]-1-pyrrolidinyl}-2-pyrimidinyl)-1H-pyrazol[3,4-b]pyridin (Beispiel XIX, Schritt 2) werden in 50 ml absolutem THF unter Argon gelöst und 2.19 g (8.37 mol) einer 1 molaren Tetrabutylammoniumfluorid-Lösung in THF zugegeben. Die Lösung wird über Nacht bei RT gerührt. Man verdünnt mit 50 ml einer 1:1-Mischung aus destilliertem Wasser und Ethylacetat und extrahiert die wässrige Phase nach Abtrennung dreimal mit Ethylacetat. Das Lösungsmittel der vereinigten organischen Phasen wird im Vakuum entfernt und das erhaltene Rohprodukt über Kieselgel chromatographiert (Eluent: DCM/Methanol 30:1). Man erhält 1.64 g (80% d.Th.) des Produktes.
HPLC: Rₜ = 4.02 min.
MS (ESIpos): m/z = 391 (M+H)⁺
R_{f} = 0.42 (DCM/Methanol 20:1)
¹H-NMR (300 MHz, DMSO-d₆): δ = 1.40 (s, 1H), 1.89-2.17 (m, 2H), 3.42-3.58 (m, 3H), 4.46 (s, 1H), 5.04 (d, 1H), 5.80 (s, 2H), 7.10-7.28 (m, 3H), 7.30-7.42 (m, 2H), 8.25 (s, 2H), 8.62 (d, 1H), 8.83 (d, 1H).

Die folgenden Beispiele XX und XXI sind durch Umsetzung der entsprechenden Halogenpyridine (4-Chlor- oder 3-Brompyridine) zugänglich, indem diese entweder analog der Vorschrift in Saji, H.; Watanabe, A.; Magata, Y.; Ohmomo Y.; Kiyono, Y.; Yamada, Y.; Iida, Y.; Yonekura, Y.; Konishi, J.; Yokoyama, A. Chem. Pharm. Bull. 1997, 45, 284-290 mit Hexabutyldistannan unter Zusatz von Pd(PPh₃)₄ in Toluol umgesetzt werden oder analog der Vorschrift aus Garg, S.; Garg, P.K;, Zalutsky, M.R.; Bioconjugate Chem. 1991, 2, 50-56 mit Buthyllithium lithiiert werden und anschließend die Einführung des Stannylrests mit Chlortributylstannan erfolgt:

### Beispiel XX

### 2-Fluor-4-(tributylstannyl)pyridin

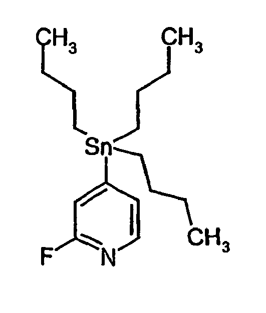

### Beispiel XXI

2-Fluor-3-(tributylsamyl)pyridin

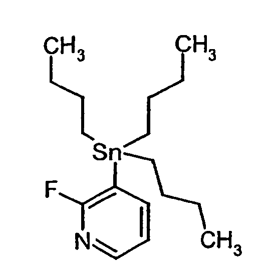

### Beispiel XXII

### 5-Cyclopropyl-4-(tributylstannyl)isoxazol

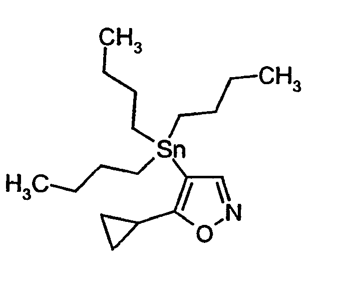

Diese Verbindung kann auf analoge Weise zu den Verbindungen aus den Beispielen XX und XXI hergestellt werden.

### Bespiel XXIII

### Ethyl 2-brom-4-fluorbenzoat

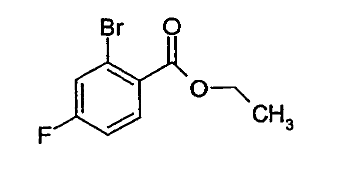

Zu einer Lösung von 220 mg (1.01 mmol) 2-Brom-4-fluorbenzoesäure in 5 ml absolutem Toluol unter Argon werden 281 mg (2.21 mmol) Oxalylchlorid gegeben und für 4 Stunden bei RT gerührt. Das Lösungsmittel wird im Vakuum entfernt und der Rückstand wird dreimal in DCM aufgenommen und das Lösungsmittel jeweils im Vakuum entfernt. Man erhält ein gelbes Öl, welches in wenig DCM gelöst wird. Man versetzt die Lösung mit 213 mg (2.10 mmol) Triethylamin und 2 ml Ethanol und rührt bei RT für 3 Stunden. Das Reaktionsgemisch wird mit dem doppelten Volumen an destilliertem Wasser verdünnt und mit DCM extrahiert. Das Lösungsmittel der organischen Phase wird im Vakuum entfernt und der Rückstand über Kieselgel chromatographiert (Eluent: DCM/Methanol 20:1). Man erhält 110 mg (39 % d.Th.) des Produktes.
HPLC: Rₜ = 4.80 min.
MS (ESIpos): m/z = 248 (M+H)⁺
¹H-NMR (200 MHz, DMSO-d₆): δ = 1.32 (t, 3H), 4.32 (q, 2H), 7.40 (t, 1H), 7.73 (dd, 1H), 7.81 (dd, 1H).

### Beispiel XXIV

### 1-(2-Fluorbenzyl)-3-[5-(trimethylstannyl)-2-pyrimidinyl]-1H-pyrazolo[3,4-b]pyridin

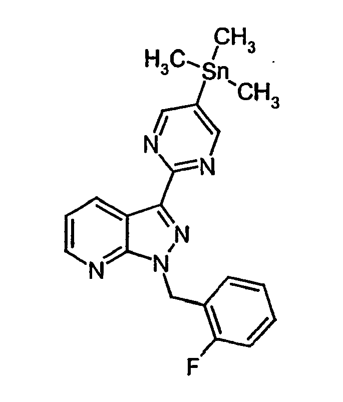

Zu einer Lösung aus 2.00 g (5.21 mmol) 3-(5-Brom-2-pyrimidinyl)-1-(2-fluorbenzyl)-1H-pyrazol[3,4-b]pyridin (Beispiel I, Schritt 6) in 75 ml absolutem Dioxan unter Argon werden 5.12 g (15.62 mmol) 1,1,1,2,2,2-Hexamethyldistannan und 0.59 g (0.83 mmol) Bis-(triphenylphosphin)palladium(II)-chlorid zugegeben und über Nacht bei 80-85°C gerührt. Man hydrolysiert durch Zugabe von 40 ml einer 1 molaren wässrigen Kaliumfluorid-Lösung und extrahiert dreimal mit Ethylacetat. Die vereinigten organischen Phasen werden über Magnesiumsulfat getrocknet, filtriert und das Lösungsmittel unter Vakuum entfernt. Das Rohprodukt wird über Kieselgel aufgereinigt (Eluent: Cyclohexan/Ethylacetat 3:1). Man erhält 1.56 g (60% d.Th.) des Produktes.
LC/MS (Methode D): Rₜ = 5.20 min.
MS (ESIpos): m/z = 469 (M+H)⁺
R_{f} = 0.75 (Toluol/Ethylacetat 1:1)
¹H-NMR (200 MHz, DMSO-d₆): δ = 0.39 (t, 9H), 5.87 (s, 2H), 7.10-7.49 (m, 5H), 8.68 (d, 1H), 8.87 (d, 1H), 8.90 (s, 3H).

### Beispiel XXV

### Ethyl 2-brom-5-fluorbenzoat

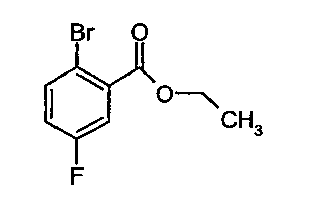

Die Verbindung wird analog der Vorschrift des Beispiels XXIII hergestellt. Ausgehend von 670 mg (3.06 mmol) 2-Brom-5-fluorbenzoesäure erhält man 320 mg (34 % d.Th.) des Produktes.
HPLC: Rₜ = 4.66 min.
MS (ESIpos): m/z = 248 (M+H)⁺
R_{f}= 0.90 (DCM/Methanol 20:1)
¹H-NMR (300 MHz, DMSO-d₆): δ = 1.33 (t, 3H), 4.33 (q, 2H), 7.37 (t, 1H), 7.61 (dd, 1H), 7.81 (dd, 1H).

### Beispiel XXVI

### Schritt 1

### 1-Cyclopropyl-2- {2-[1-(2-fluorbenzyl)-1H-pyrazolo[3,4-b]pyridin-3-yl]-5-pyrimidinyl}ethanon

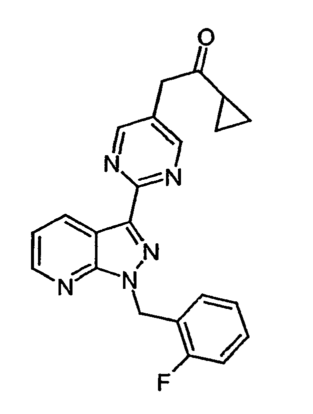

500 mg (5.2 mmol) gut getrocknetes Natrium-tert-butylat werden in einer ausgeheizten und evakuierten Apparatur unter Argon vorgelegt. 143 mg (0.16 mmol) Tris-(dibenzylidenaceton)dipalladium, 243 mg (0.39 mmol) rac-BINAP und 1000 mg (2.6 mmol) 3-(5-Brom-2-pyrimidinyl)-1-(2-fluorbenzyl)-1H-pyrazol[3,4-b]pyridin (Beispiel I, Schritt 6) werden nacheinander zugegeben und die Apparatur nochmals evakuiert und mit Argon gespült. Die Reagenzien werden in 40 ml absolutem Dioxan suspendiert, mit 438 mg (0.47 ml, 5.2 mmol) Cyclopropylmethylketon versetzt und 2 Stunden auf Rückfluss erhitzt. Das Lösungsmittel wird in Vakuum entfernt und der Rückstand mittels Chromatographie auf Kieselgel (Eluent: DCM/Methanol 20:1) gereinigt. Man erhält 630 mg (57% d.Th.) des Produktes.
HPLC: Rₜ = 4.53 min.
MS (ESIpos): m/z = 388 (M+H)⁺
R_{f} = 0.51 (DCM/Methanol 20:1)
¹H-NMR (300 MHz, DMSO-d₆) : δ = 0.92-1.02 (m, 4H), 2.20 (quintett, 1H), 4.12 (s, 2H), 5.87 (s, 2H), 7.16 (q, 1H), 7.22-7.49 (m, 3H), 7.42 (dd, 1H), 8.67 (d, 1H), 8.77 (s, 2H), 8.88 (d, 1H).

### Schritt 2

### (2E)-1-Cyclopropyl-3-(dimethylamino)-2-{2-[1-(2-fluorbenzyl)-1H-pyrazol[3,4-b]-pyridin-3-yl]-5-pyrimidinyl}-2-propen-1-on

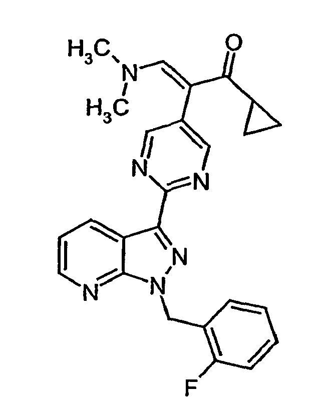

Zu einer Lösung von 123 mg (1.03 mmol) N-(Dimethoxymethyl)-N,N-dimethylamin in 1 ml DMF werden 100 mg (0.26 mmol) 1-Cyclopropyl-2-{2-[1-(2-fluorbenzyl)-1H-pyrazolo[3,4-b]pyridin-3-yl]-5-pyrimidinyl}ethanon (Beispiel XXVI, Schritt 1) gegeben. Das Reaktionsgemisch wird für 2 Stunden bei 110°C gerührt und dann in destilliertes Wasser gegossen. Der ausgefallene Feststoff wird abfiltriert, mit destilliertem Wasser gewaschen und getrocknet. Man erhält 93 mg (64% d.Th.) des Produktes, welches ohne weitere Aufreinigung direkt weiter umgesetzt wird.
HPLC: Rₜ = 4.67 min.
MS (ESIpos): m/z = 443 (M+H)⁺
¹H-NMR (200 MHz, DMSO-d₆): δ = 0.67-0.75 (m, 2H), 0.78-0.84 (m, 2H), 2.13-2.31 (m, 1H), 2.83 (s, 6H), 5.86 (s, 2H), 7.10-7.46 (m, 5H), 7.94 (s, 1H), 8.66 (s, 3H), 8.90 (d, 1H).

### Ausführungsbeispiele:

### Beispiel 1

### 1-(2-Fluorbenzyl)-3-{5-[(1S,5R)-1,3,3-trimethyl-6-azabicyclo[3.2.1]oct-6-yl]-2-pyrimidinyl}-1H-pyrazol[3,4-b]pyridin

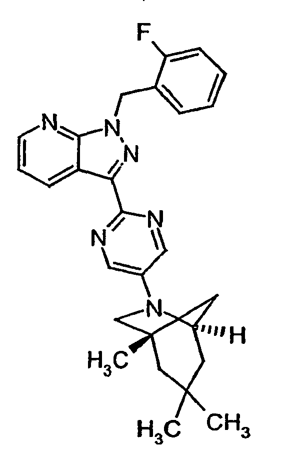

95 mg (0.85 mmol) gut getrocknetes Kalium-tert-butylat werden in einer ausgeheizten und evakuierten Apparatur unter Argon vorgelegt. 18 mg (0.02 mmol) Tris-(dibenzylidenaceton)dipalladium, 48 mg (0.08 mmol) rac-BINAP und 300 mg (0.77 mmol) 3-(5-Brom-2-pyrimidinyl)-1-(2-fluorbenzyl)-1H-pyrazolo[3,4-b]pyridin (Beispiel I, Schritt 6) werden nacheinander zugegeben und die Apparatur nochmals evakuiert und mit Argon gespült. Die Reagenzien werden in 4.5 ml absolutem Toluol suspendiert und dann gibt man 355 mg (2.32 mmol) (1S,5R)-1,3,3-Trimethyl-6-azabicyclo[3.2.1]octan zu der Reaktionsmischung. Diese rührt man bei 60°C über Nacht. Das Lösungsmittel wird in Vakuum entfernt und der Rückstand mittels präparativer HPLC gereinigt. Man erhält 73 mg (20% d.Th.) des Produktes.
LC/MS (Methode A): Rₜ = 3.79 min.
MS (ESIpos): m/z = 457 (M+H)⁺
R_{f}= 0.75 (DCM/Methanol 20:1)
¹H-NMR (300 MHz, DMSO-d₆): δ = 0.74 (s, 3H), 0.92 (s, 3H), 1.15 (s, 3H), 1.34 (d, 1H), 1.45-1.61 (m, 3H), 1.78 (br s, 1H), 1.91 (d, 1H), 3.18 (dd, 2H), 4.28 (br s, 1H), 5.79 (s, 2H), 7.09-7.26 (m, 4H), 7.30-7.42 (m, 1H), 8.21 (s, 2H), 8.61 (d, 1H), 8.82 (d, 1H).

Die in der folgenden Tabelle aufgeführten Beispiele können analog der Vorschrift des Beispiels 1 aus den entsprechenden Ausgangsverbindungen hergestellt werden:

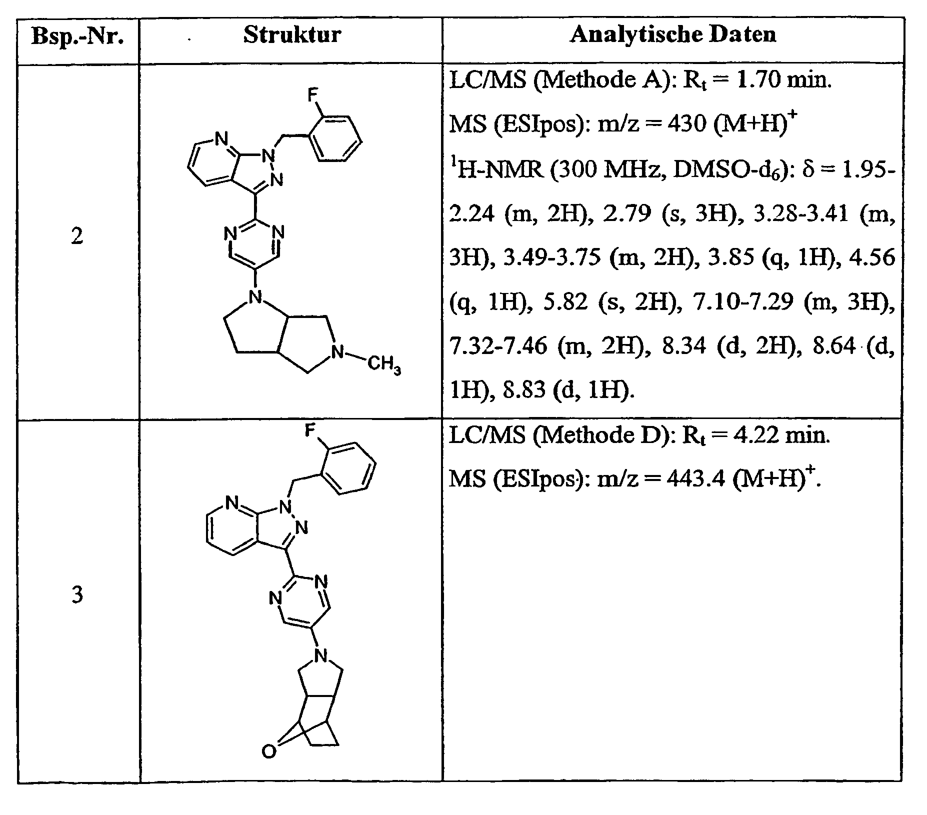

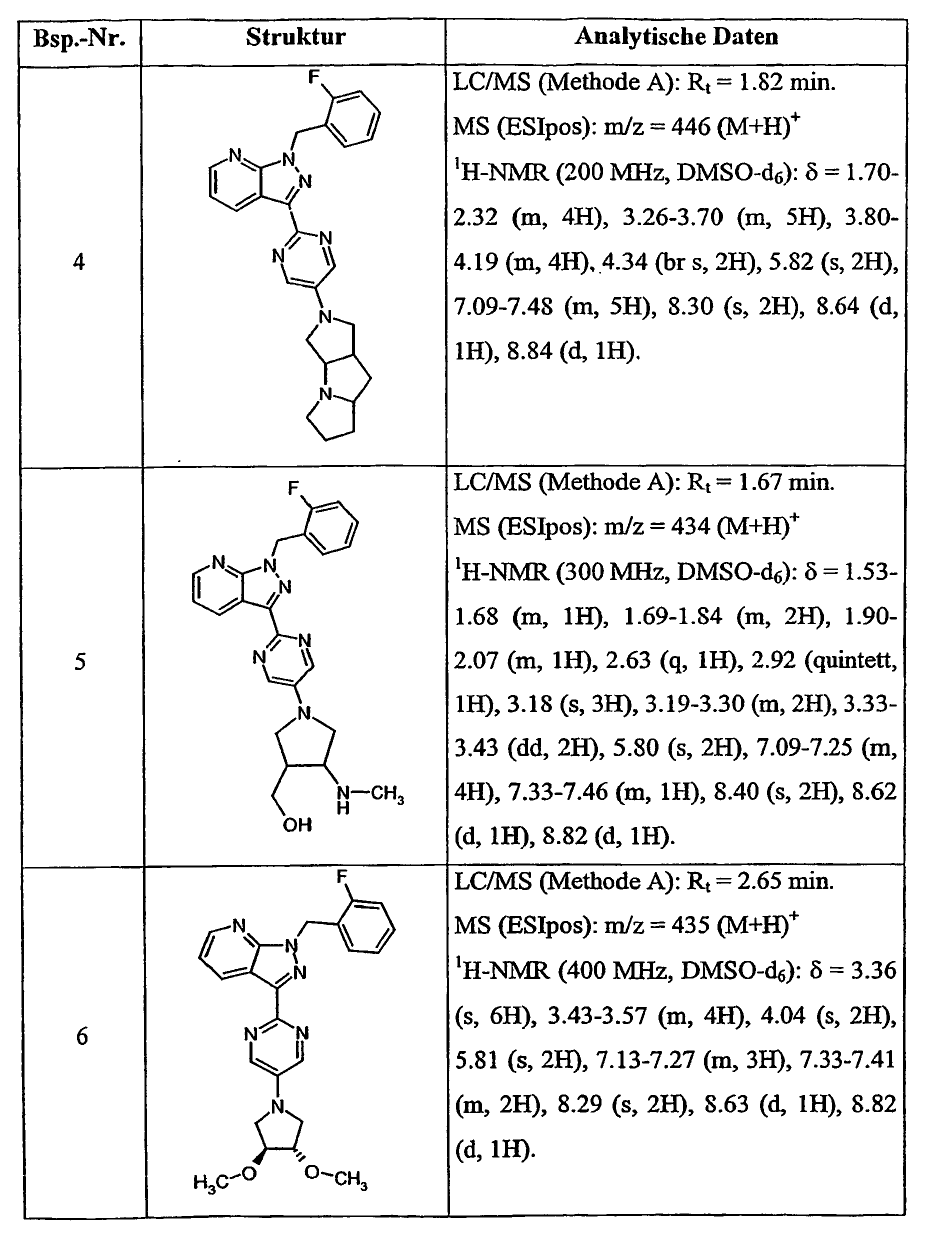

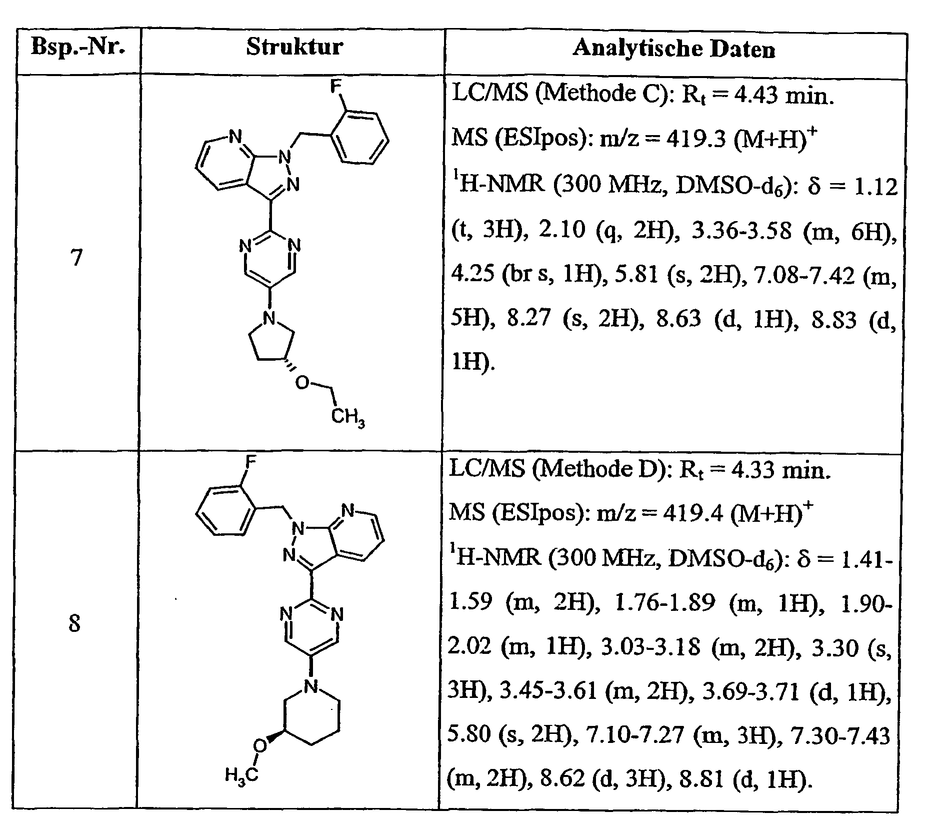

### Beispiel 9

### 3-[5-(2,5-Diazabicyclo[2.2.1]hept-2-yl)-2-pyrimidinyl]-1-(2-fluorbenzyl)-1H-pyrazol-[3,4-b]pyridin

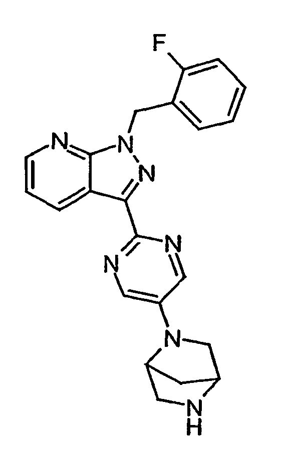

250 mg (0.50 mmol) tert-Butyl-5-{2-[1-(2-fluorbenzyl)-1H-pyrazolo[3,4-b]pyridin-3-yl]-5-pyrimidinyl}-2,5-diazabicyclo-[2.2.1]-heptan-2-carboxylat (Beispiel VI, Schritt 4) werden in 2 ml DCM gelöst und mit 2 ml TFA versetzt. Man rührt für eine Stunde bei RT. Man verdünnt mit DCM und stellt die Lösung mit wässriger Natriumcarbonat-Lösung basisch. Die organische Phase wird abgetrennt, mit gesättigter wässriger Natriumchlorid-Lösung gewaschen und getrocknet. Das Lösungsmittel wird im Vakuum entfernt und man erhält 163 mg (82% d.Th.) des Produktes.
LC/MS (Methode D): Rₜ = 2.65 min.
MS (ESIpos): m/z = 402.5 (M+H)⁺
¹H-NMR (300 MHz, DMSO-d₆): δ =1.76 (dd, 2H), 2.85 (dd, 2H), 3.07 (d, 1H), 3.56 (d, 1H), 3.69 (s, 1H), 4.59 (s, 1H), 5.80 (s, 2H), 7.09-7.28 (m, 3H), 7.30-7.43 (m, 2H), 8.30 (s, 2H), 8.62 (d,1H), 8.81 (d, 1H).

Die in der folgenden Tabelle aufgeführten Beispiele können analog der Vorschrift des Beispiels 9 aus den entsprechenden Ausgangsverbindungen hergestellt werden:

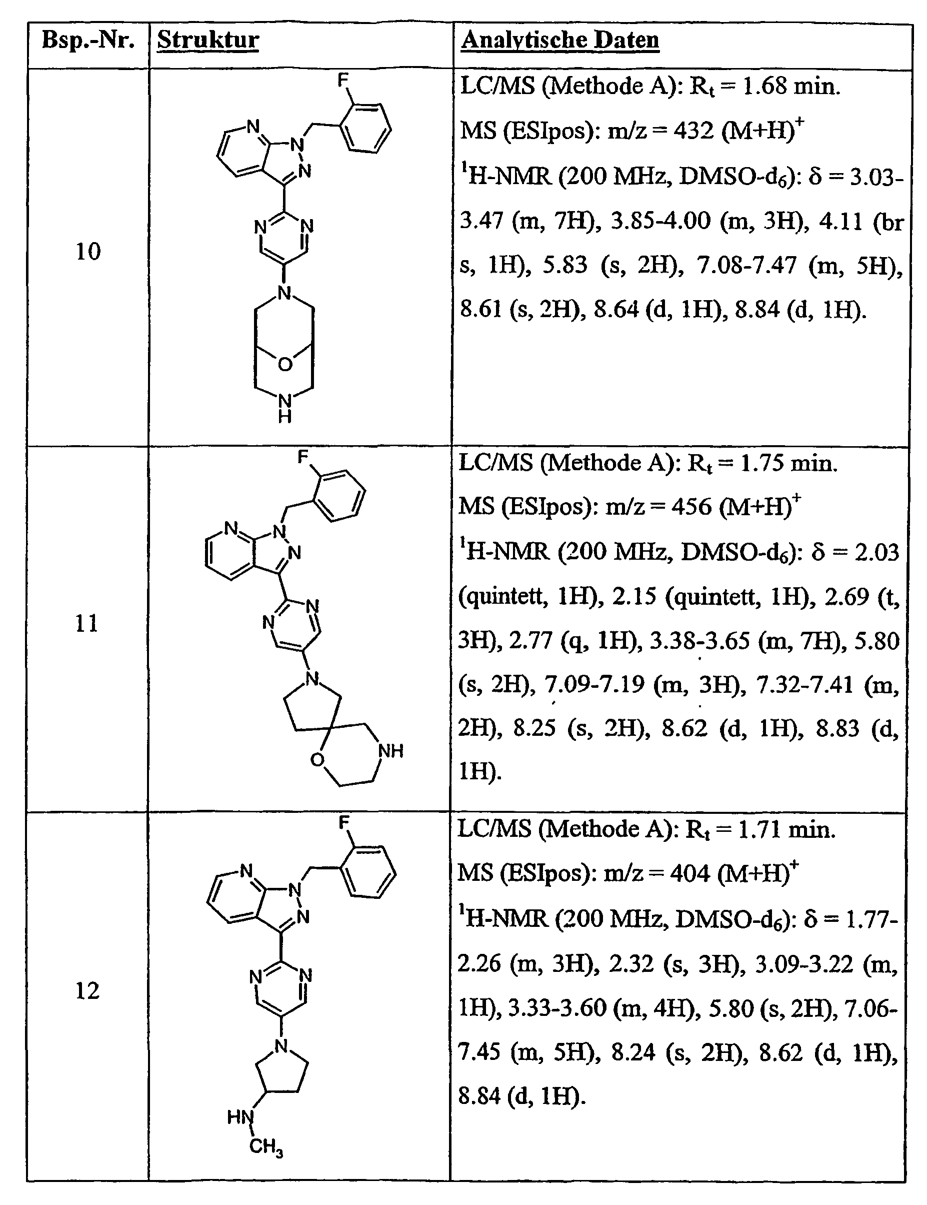

### Beispiel 13

### 2-{2-[1-(2-Fluorbenzyl)-1H-pyrazol[3,4-b]pyridin-3-yl]-5-pyrimidinyl}cyclohexanon

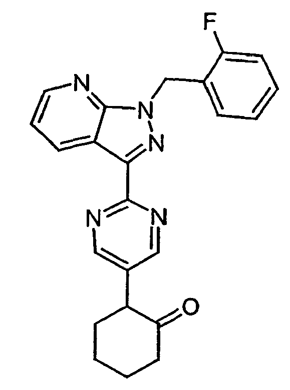

Die Verbindung wird bis auf die folgenden Veränderungen analog der Vorschrift des Beispiels 1 hergestellt. Die Reaktion wird in absolutem Dioxan mit Natrium-tert-butylat anstatt der entsprechenden Kalium-Verbindung und bei 70°C durchgeführt. Ausgehend von 100 mg (0.26 mmol) 3-(5-Brom-2-pyünüdulyl)-1-(2-fluorbenzyl)-1H-pyrazol[3,4-b]pyridin (Beispiel I, Schritt 6) und 77 mg (0.78 mmol) Cyclohexanon erhält man 31 mg (29% d.Th.) des Produktes.
LC/MS (Methode D): Rₜ = 4.26 min.
MS (ESIpos): m/z = 402.3 (M+H)⁺
R_{f} = 0.40 (Toluol/Ethylacetat 1:1)
¹H-NMR (200 MHz, DMSO-d₆): δ = 1.70-2.43 (m 7H), 2.54-2.72 (m, 1H), 3.99 (dd, 1H), 5.87 (s, 2H), 7.11-7.50 (m, 5H), 8.68 (d, 1H), 8.70 (s, 2H), 8.89 (d, 1H).

Das in der folgenden Tabelle aufgeführte Beispiel kann analog der Vorschrift des Beispiels 13 aus der entsprechenden Ausgangsverbindung hergestellt werden:

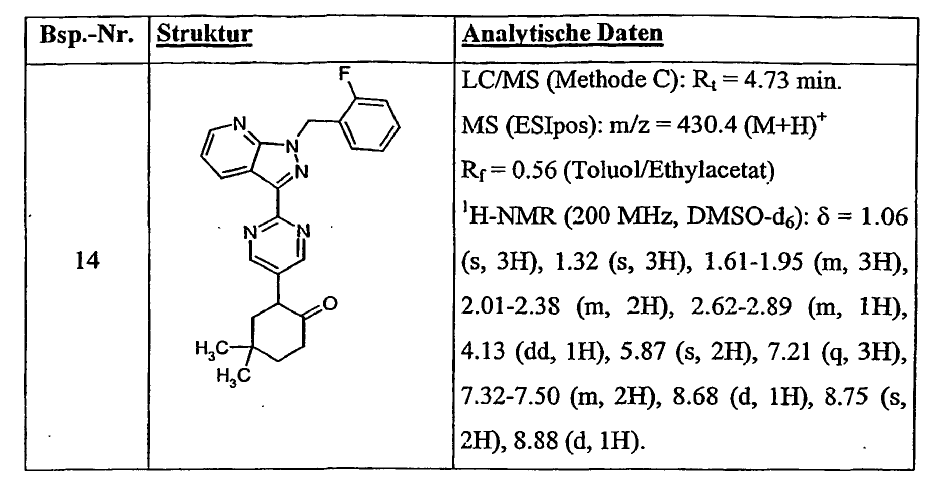

### Beispiel 15

### 1-(2-Fluorbenzyl)-3-[5-(5-isopropyl-2,5-diazabicyclo[2.2.1]hept-2-yl)-2-pyrimidinyl]-1H-pyrazol[3,4-b]pyridin

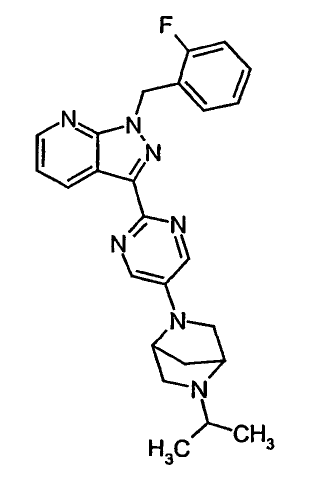

50 mg (0.13 mmol) 3-[5-(2,5-Diazabicyclo[2.2.1]hept-2-yl)-2-pyrimidinyl]-1-(2-fluorbenzyl)-1H-pyrazolo[3,4-b]pyridin (Beispiel 9) werden in 5 ml absolutem Aceton gelöst und mit 92 mg (0.87 mmol) Natriumcarbonat versetzt. Zu der Suspension gibt man 64 mg (0.37 mmol) 2-Iodpropan und rührt bei RT über Nacht. Die Reaktionslösung wird mit Wasser versetzt und mit DCM extrahiert. Die organischen Phasen werden vereinigt, mit gesättigter wässriger Natriumchlorid-Lösung gewaschen, getrocknet und filtriert. Das Lösungsmittel wird im Vakuum entfernt und der Rückstand mittels präparativer HPLC unter Zusatz geringer Anteile wässriger Salzsäure gereinigt. Man erhält 25 mg (42% d.Th.) des Produktes.
LC/MS (Methode A): Rₜ = I .78 min.
MS (ESIpos): m/z = 444 (M+H)⁺.

### Beispiel 16

### 5-{2-[1-(2-Fluorbenzyl)-1H-pyrazolo[3,4-b]pyridin-3-yl]-5-pyrimidinyl}-2,2-dimethyl-5-aza-2-azoniabicyclo[2.2.1]heptanchlorid

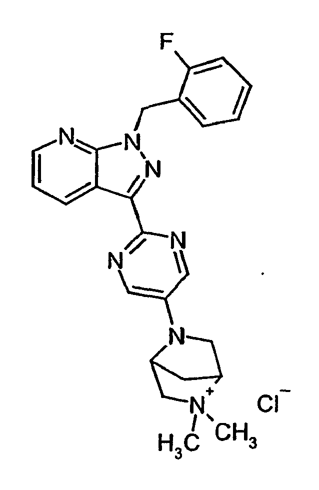

Die Verbindung wird analog der Vorschrift für Beispiel 15 mit den entsprechenden Ausgangsmaterialien hergestellt. Man erhält 51 mg (88 % d.Th.) des Produktes.
LC/MS (Methode A): Rt = 1.65 min.
MS (ESIpos): m/z = 430 (M+H)⁺
¹H-NMR (200 MHz, DMSO-d₆): δ = 2.34 (d, 1H), 2.71 (d, 1H), 3.11 (s, 3H), 3.28 (s, 3H), 3.62-3.76 (m, 3H), 4.64 (s, 1H), 4.90 (s, 1H), 5.82 (s, 2H), 7.11-7.29 (m, 3H), 7.32-7.44 (m, 2H), 8.44 (s, 2H), 8.63 (d, 1H), 8.82 (d, 1H).

### Beispiel 17

### 1-(2-Fluorbenzyl)-3-[5-(5-methyl-2,5-diazabicyclo[2.2.1]hept-2-yl)-2-pyrimidinyl-1H-pyrazolo[3,4-b]pyridin

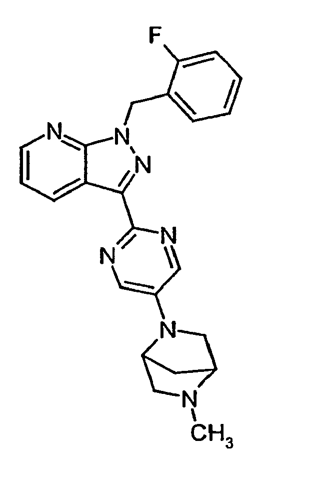

40 mg (0.10 mmol) 3-[5-(2,5-Diazabieyclo[2.2.1]hept-2-yl)-2-pyrimidinyl]-1-(2-fluorbenzyl)-1H-pyrazolo[3,4-b]pyridin (Beispiel 9) werden in 0.5 ml absolutem DMF gelöst. Zu dieser Lösung gibt man 542 mg (6.67 mmol) wässrige Formaldehyd-Lösung (37%-ig) und 1220 mg (26.51 mmol) Ameisensäure und erhitzt das Reaktionsgemisch für 16 Stunden auf 80°C. Die Reaktionslösung wird mit 1 molarer Natriumhydroxid-Lösung basisch gestellt und mit DCM extrahiert. Die organische Phase wird mit gesättigter wässriger Natriumchlorid-Lösung gewaschen, getrocknet und das Lösungsmittel im Vakuum entfernt. Man erhält 39 mg (94 % d.Th.) des Produktes.
HPLC: Rₜ = 3.39 min.
LC/MS (Methode C): Rₜ = 2.61 min.
MS (ESIpos): m/z = 416.24 (M+H)⁺
¹H-NMR (400 MHz, DMSO-d₆): δ = 1.78 (d, 1H), 1.93 (d, 1H), 2.28 (s, 3H), 2.73 (s, 1H), 2.81 (d, 1H), 2.89 (s, 1H), 3.32 (d, 1H), 3.38 (d, 1H), 3.51 (s, 1H), 5.81 (s, 2H), 7.10-7.28 (m, 3H), 7.32-7.43 (m, 2H), 8.32 (s, 2H), 8.62 (d, 1H), 8.81 (d, 1H).

### Beispiel 18

### 2- {2-[1-(2-Fluorbenzyl)-1H-pyrazolo[3,4-b]pyridin-3-yl]-5-pyrimidinyl}-9-methyl-6-oxa-2,9-diazaspiro[4.5]decan-Hydrochlorid

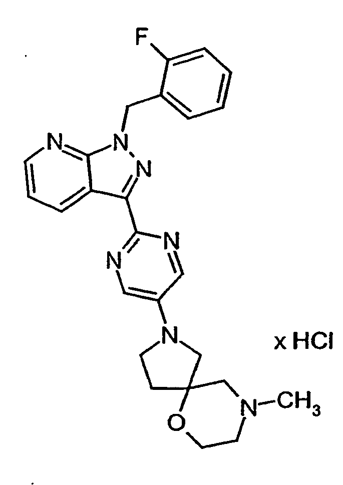

Die Verbindung wird analog der Vorschrift für Beispiel 17 aus Beispiel 11 hergestellt. Man erhält 49 mg (88 % d.Th.) des Produktes. Bei der Reinigung durch HPLC unter Zusatz von geringen Anteilen Salzsäure bildet sich das Hydrochlorid.
LC/MS (Methode D): Rₜ = 2.78 min.
MS (ESIpos): m/z = 460.4 (M+H)⁺
¹H-NMR (300 MHz, DMSO-d₆): δ = 2.09-2.40 (m, 2H), 2.57-2.74 (m, 1H), 2.80 (s, 3H), 3.00-3.25 (m, 2H), 3.29-3.70 (m, 6H), 3.80-4.03 (m, 3H), 5.81 (s, 2H), 7.10-7.29 (m, 3H), 7.33-7.45 (m, 2H), 8.26 (d, 2H), 8.63 (d, 1H), 8.84 (t, 1H), 11.20 (br s, 1H).

### Beispiel 19

### 1-(2-Fluorbenzyl)-3-[5-(8-oxa-3-azabicyclo[3.2.1]oct-3-yl)-2-pyrimidinyl]-1H-pyrazolo[3,4-b]pyridin

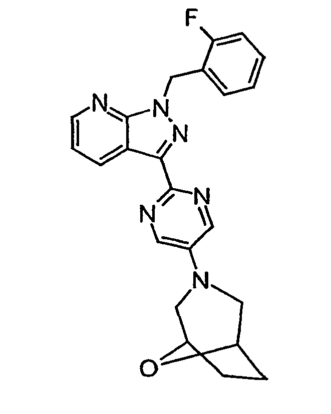

400 mg (0.68 mmol) 3-[4,6-Dichlor-5-(8-oxa-3-azabieyclo[3.2.1]oct-3-yl)-2-pyrimidinyl]-1-(2-fluorbenzyl)-1H-pyrazolo[3,4-b]pyridin (Beispiel XII, Schritt 7) werden in 200 ml Methanol gelöst und mit 86 mg Ammoniumformiat versetzt. Unter Argon gibt man 26 mg Palladium auf Aktivkohle (10%-ig) zu und erhitzt für 3 Tage unter Rückfluss. Das Reaktionsgemisch wird abfiltriert und die Rückstände mit Methanol gewaschen. Das Lösungsmittel wird im Vakuum entfernt und der Rückstand mittels präparativer HPLC aufgereinigt. Man erhält 99 mg (35% d.Th.) des Produktes.
LC/MS (Methode D): Rₜ = 3.97 min.
MS (ESIpos): m/z = 417 (M+H)⁺
¹H-NMR (200 MHz, DMSO-d₆): δ = 1.88 (s, 4H), 2.98 (dd, 2H), 3.61 (d, 1H), 4.49 (s, 2H), 5.82 (s, 2H), 7.09-7.46 (m, 5H), 8.53 (s, 2H), 8.64 (dd, 1H), 8.82 (dd, 1H).

### Beispiel 20

### 1-(2-Fluorbenzyl)-3- {5-[(3S)-3-methoxy-1-pyrrolidinyl]-2-pyrimidinyl}-1H-pyrazolo[3,4-b]pyridin

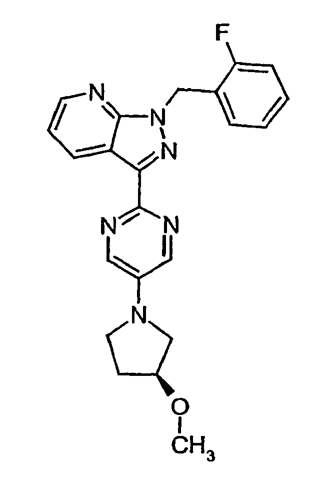

100 mg (0.21 mmol, Reinheit 80%) (3S)-1-{2-[1-(2-Fluorbenzyl)-1H-pyrazolo[3,4-b]-pyridin-3-yl]-5-pyrimidinyl}-3-pyrrolidinol (Beispiel XIX, Schritt 3) werden unter Argon in 2 ml DMF gelöst und unter Eisbadkühlung mit 8 mg (0.33 mmol) Natriumhydrid versetzt. Zu dieser Suspension tropft man ebenfalls unter Eisbad-Kühlung 32 mg (0.23 mmol) Iodmethan und rührt 2 Stunden bei dieser Temperatur nach. Man gibt 0.5 ml destilliertes Wasser zu und chromatographiert das Reaktionsgemisch direkt über eine präparative HPLC. Man erhält 35 mg (42 % d.Th.) des Produktes.
LC/MS (Methode D): Rₜ = 4.12 min.
MS (ESIpos): m/z = 405.4 (M+H)⁺
¹H-NMR (300 MHz, DMSO-d₆): δ = 2.05-2.18 (m, 2H), 3.29 (s, 3H), 3.34-3.57 (m, 4H), 4.14 (br s, 1H), 5.80 (s, 2H), 7.10-7.28 (m, 3H), 7.29-7.43 (m, 2H), 8.26 (s, 2H), S.61 (d, 1H), δ.81 (d, 1H).

Die in der folgenden Tabelle aufgeführten Beispiele können analog der Vorschrift des Beispiels 20 aus den entsprechenden Ausgangsverbindungen hergestellt werden:

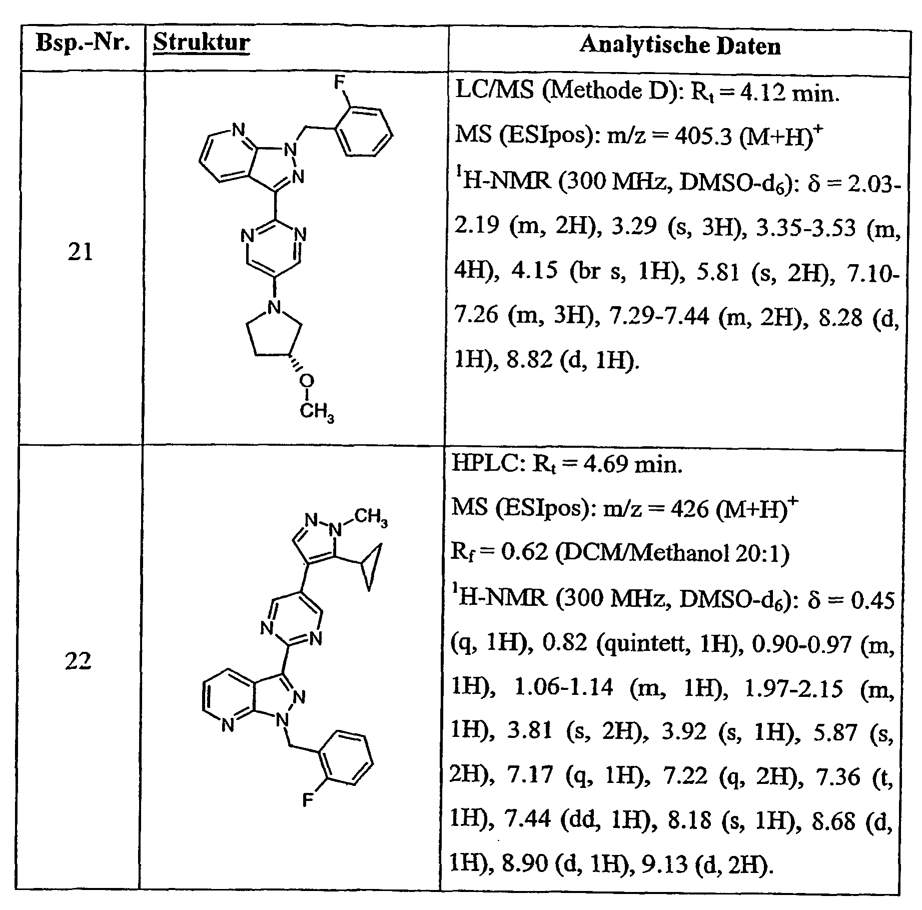

### Beispiel 23

### 3-[5-(1,3-Benzodioxol-5-yl)-2-pyrimidinyl]-1-(2-fluorbenzyl)-1H-pyrazolo[3,4-b]-pyridin

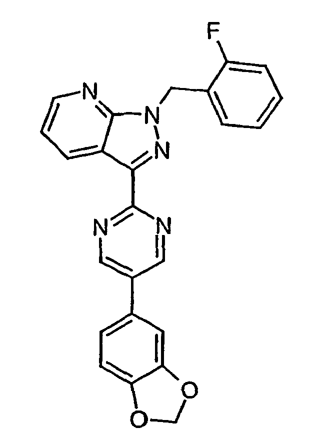

120 mg (0.31 mmol) 3-(5-Brom-2-pylimidinyl)-1-(2-fluorbenzyl)-1H-pyrazolo[3,4-b]pyridin (Beispiel I, Schritt 6) werden unter Argon in 10 ml DME gelöst und mit 67 mg (0.41 mmol) 1,3-Benzodioxol-5-yl-boronsäure versetzt. Man erwärmt auf 40°C, bis eine Lösung entstanden ist, und gibt dann 17 mg (0.02 mmol) Tetrakis(triphenylphosphin)-palladium(0) zu. Man rührt für eine Stunde unter Rückfluss, gibt nun 39 mg (0.34 mmol) Kalium-tert-butylat zu und rührt anschließend bei 85°C über Nacht. Man verdünnt mit DCM und extrahiert jeweils einmal mit 1 molarer Salzsäure und gesättigter wässriger Natriumhydrogencarbonat-Lösung. Die organische Phase wird über Magnesiumsulfat getrocknet, filtriert und das Lösungsmittel im Vakuum entfernt. Der Rückstand wird mittels präparativer HPLC aufgereinigt. Man erhält 60 mg (92% d.Th.) des Produktes.
LC/MS (Methode D): Rₜ = 4.69 min.
MS (ESIpos): m/z = 426.2 (M+H)⁺
R_{f} = 0.77 (Toluol/Ethylacetat 1:1)
¹H-NMR (300 MHz, DMSO-d₆): δ = 5.87 (s, 2H), 6.11 (s, 2H), 7.05-7.39 (m, 5H), 7.44 (q, 1H), 7.51 (d, 1H), 7.54-7.68 (m, 1H), 8.68 (d, 1H), 8.90 (d, 1H), 9.22 (s, 2H).

Die in der folgenden Tabelle aufgeführten Beispiele können analog der Vorschrift des Beispiels 23 aus den entsprechenden Ausgangsverbindungen hergestellt werden:

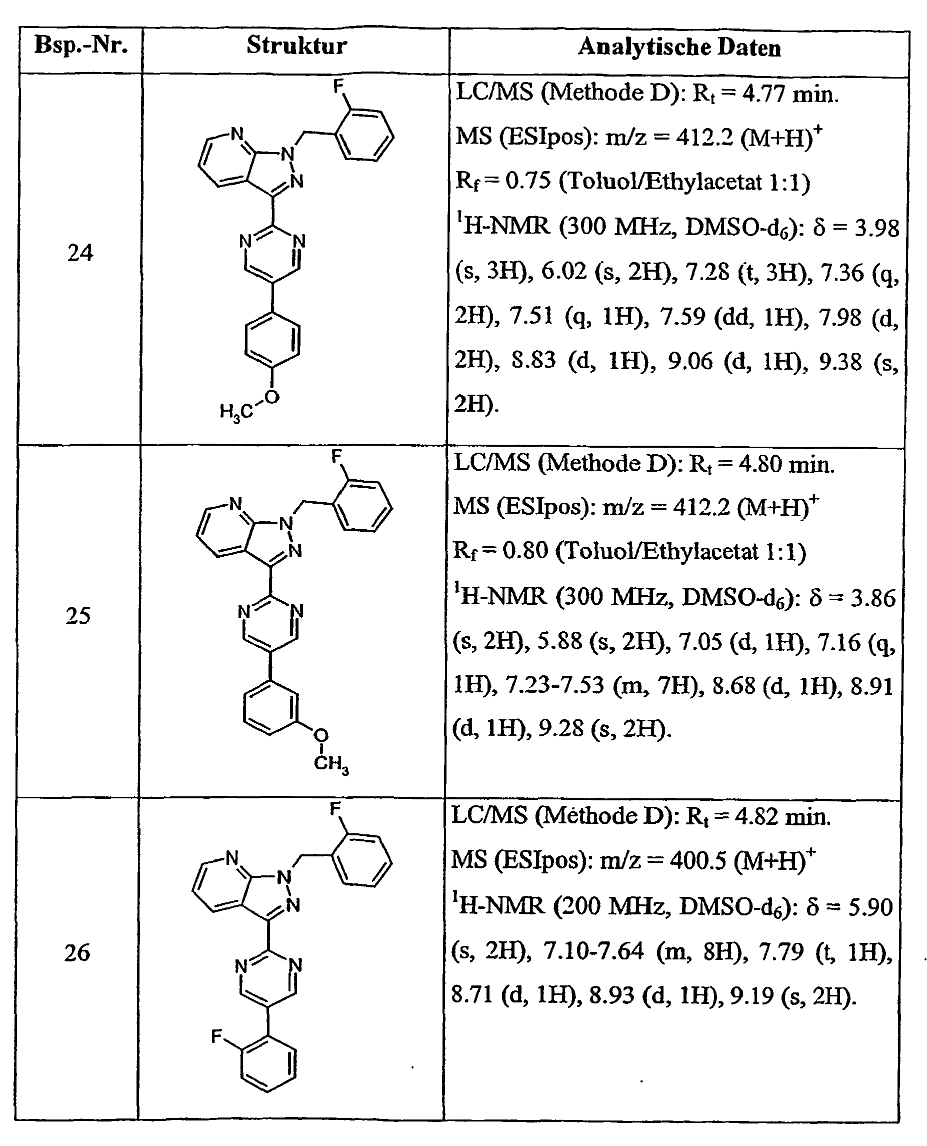

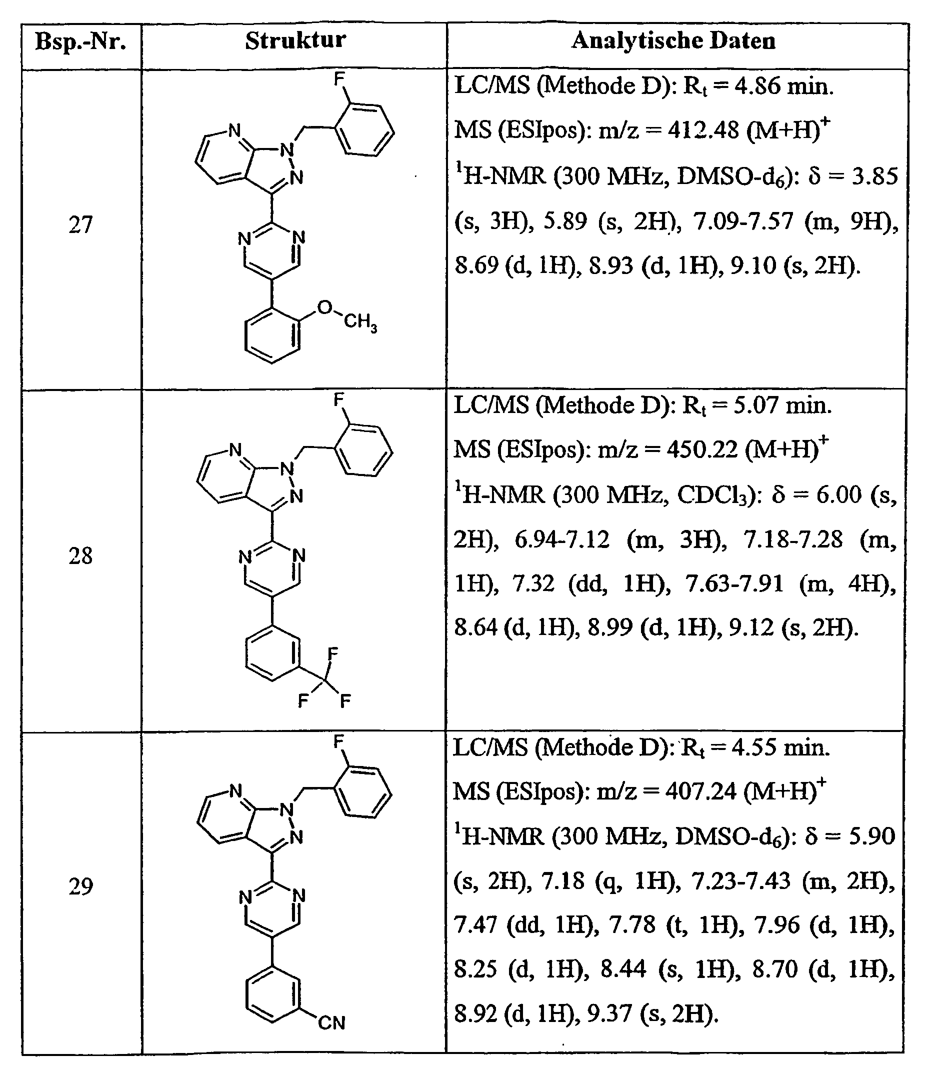

### Beispiel 30

### (2-{2-[1-(2-Fluorbenzyl)-1H-pyrazolo[3,4-b]pyridin-3-yl]-5-pyrimidinyl}phenyl)-methanol

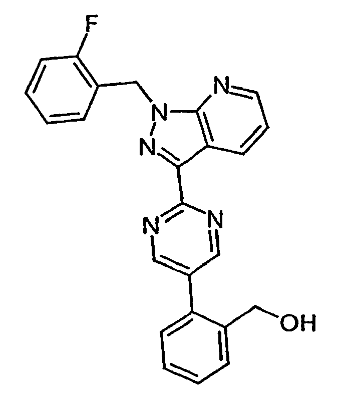

200 mg (0.52 mmol) 3-(5-Brom-2-pyrimidinyl)-1-(2-fluorbenzyl)-1H-pyrazolo[3,4-b]pyridin (Beispiel I, Schritt 6), 90 mg (0.62 mmol) 2-(Hydroxymethyl)phenylboronsäure, 40 mg (0.05 mmol) 1,1'-Bis(diphenylphosphino)-ferrocenpalladium(II)-chlorid und 200 mg (0.62 mmol) Cäsiumcarbonat werden in 2 ml DME aufgenommen und über Nacht unter Rückfluss gerührt. Das Lösungsmittel wird unter Vakuum entfernt und der Rückstand mittels präparativer HPLC aufgereinigt, so dass man 111 mg (52 % d.Th.) des Produktes erhält.
HPLC: Rₜ = 4.53 min.
LC/MS (Methode C): Rₜ = 4.17 min.
MS (ESIpos): m/z = 412 (M+H)⁺
¹H-NMR (200 MHz, CDCl₃): δ = 1.26 (t, 1H), 4.65 (d, 2H), 6.00 (s, 2H), 6.92-7.70 (m, 9H), 8.64 (d, 1H), 9.00 (s, 2H), 9.03 (d, 1H).

### Beispiel 31

### 1-(2-Fluorbenzyl)-3-[5-(2-fluor-4-pyridinyl)-2-pyrimidinyl]-1H-pyrazolo[3,4-b]-pyridin

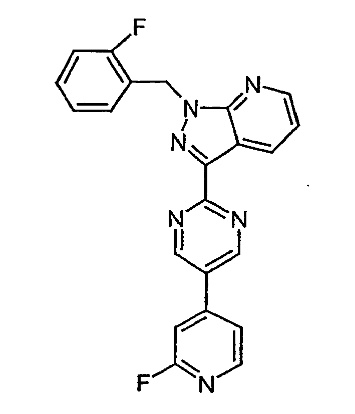

120 mg (0.31 mmol) 3-(5-Brom-2-pyrimidinyl)-1-(2-fluorbenzyI)-1H-pyrazolo[3,4-b]pyridin (Beispiel I, Schritt 6) werden unter Argon in 10 ml DMF gelöst und mit 121 mg (0.31 mmol) 2-Fluor-4-(tributylstannyl)pyridin (Beispiel XX) und 13 mg (0.02 mmol) Dichlorbis-(triphenylphosphin)palladium versetzt. Das Reaktionsgemisch wird über Nacht bei 110°C gerührt. Man verdünnt mit DCM und extrahiert mit gesättigter wässriger Ammoniumchlorid-Lösung. Die organische Phase wird über Magnesiumsulfat getrocknet, filtriert und das Lösungsmittel im Vakuum entfernt. Der Rückstand wird mittels präparativer HPLC aufgereinigt. Man erhält 41 mg (32 % d.Th.) des Produktes.
LC/MS (Methode D): Rₜ = 4.17 min.
MS (ESIpos): m/z = 401.25 (M+H)⁺
R_{f}= 0.67 (Toluol/Ethylacetat 2:1)
¹H-NMR (300 MHz, DMSO-d₆): δ = 5.91 (s, 2H), 7.18 (q, 1H), 7.22-7.43 (m, 3H), 7.48 (dd, 1H), 7.91 (t, 1H), 8.63 (d, 1H), 8.71 (d, 1H), 8.80 (d, 1H), 8.93 (d, 1H), 9.30 (s, 2H).

Die in der folgenden Tabelle aufgeführten Beispiele können analog der Vorschrift des Beispiels 31 aus den entsprechenden Ausgangsverbindungen hergestellt werden:

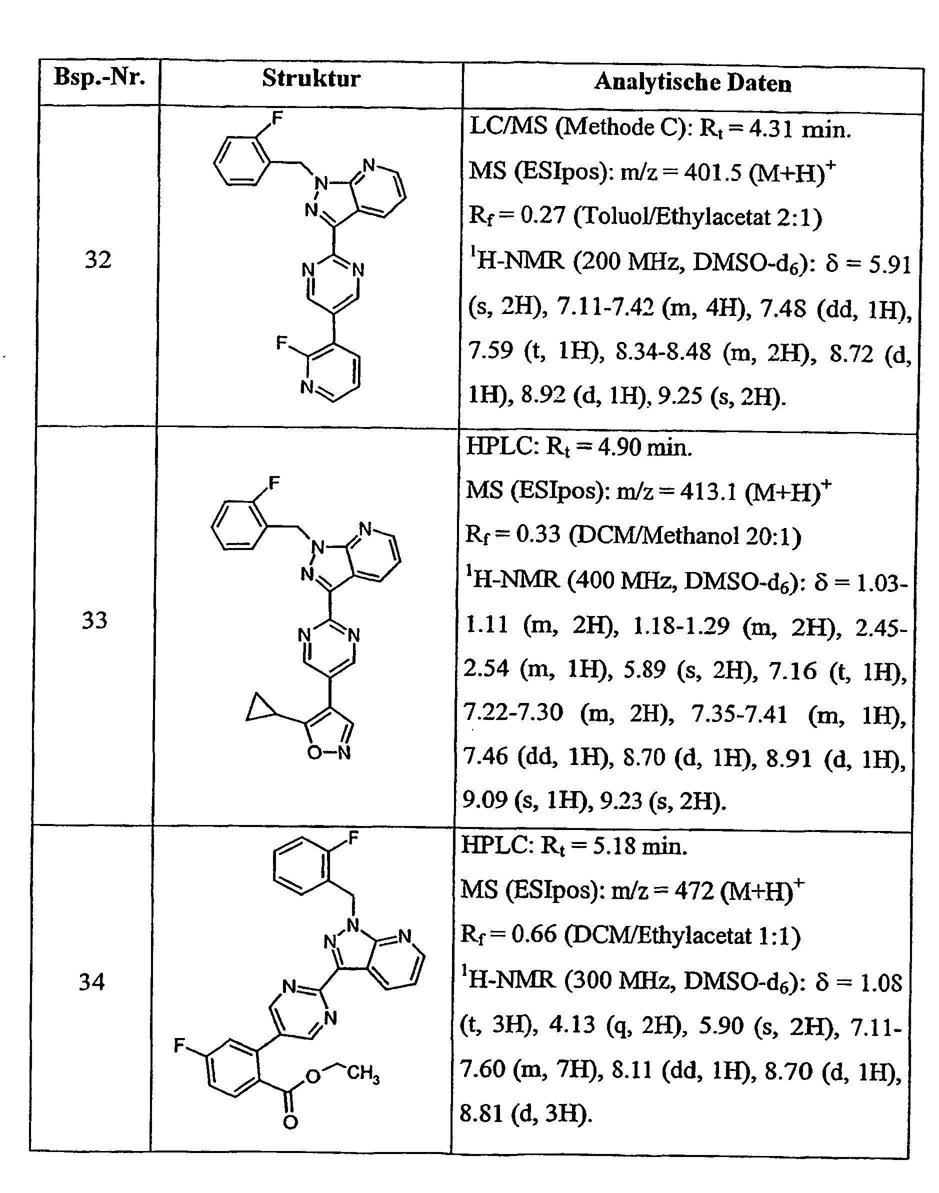

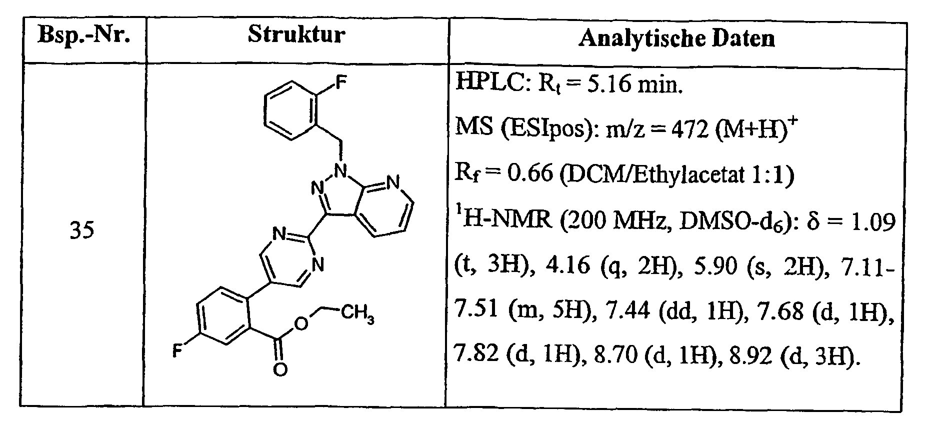

### Beispiel 36

### 3-[5-(5-Cyclopropyl-1H-pyrazol-4-yl)-2-pyrimidinyl]-1-(2-fluorbenzyl)-1H-pyrazolo[3,4-b]pyridin

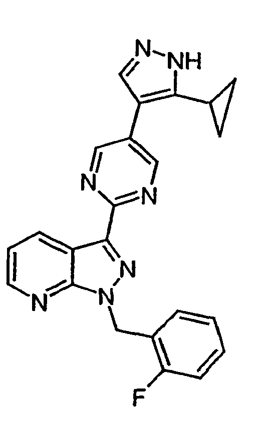

90 mg (0.20 mmol) (2E)-1-Cyclopropyl-3-(dimethylamino)-2-{2-[1-(2-fluorbenzyl)-1H-pyrazolo[3,4-b]pyridin-3-yl]-5-pyrimidinyl}-2-propen-1-on (Beispiel XXVI, Schritt 2) werden unter Argon in 3 ml absolutem Methanol gelöst. Man gibt 102 mg (2.03 mmol) Hydrazinhydrat zu und rührt 2 Stunden unter Rückfluss. Das Lösungsmittel wird im Vakuum entfernt und der Rückstand mittels präparativer HPLC aufgereinigt. Man erhält 48 mg (58 % d.Th.) des Produktes.
HPLC: Rₜ = 4.37 min.
MS (ESIpos): m/z=412 (M+H)⁺
R_{f}= 0.36 (DCM/Methanol 20:1).

### Beispiel 37

### 3- {5-[5-Cyclopropyl-1-(2,2,2-trifluorethyl)-1H-pyrazol-4-yl]-2-pyrimidinyl}-1-(2-fluorbenzyl)-1H-pyrazolo[3,4-b]pyridin

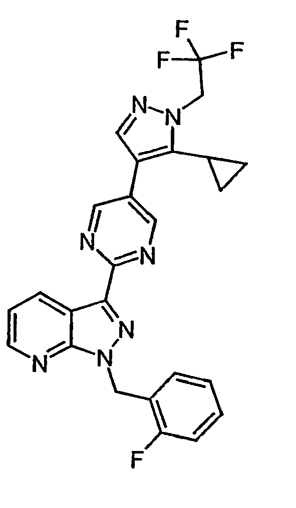

Die Verbindung wird analog der Vorschrift für Beispiel 36 unter Verwendung von 2,2,2-Trifluorethylhydrazin hergestellt. Die Ausbeute des Produktes liegt bei 9 % d.Th.
LC/MS (Methode A): Rₜ = 2.87 min.
MS (ESIpos): m/z = 494 (M+H)⁺
R_{f} = 0.70 (DCM/Methanol 20:1).

### Beispiel 38

### 1-{2-[1-(2-Fluorbenzyl)-1H-pyrazol[3,4-b]pyridin-3-yl]-5-pyrimidinyl}-3-pyrrolidinon

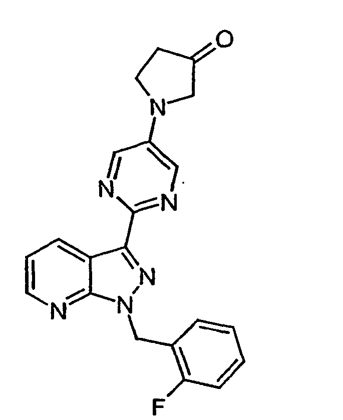

100 mg (0.26 mmol) (3R)-1-{2-[1-(2-Fluorbenzyl)-1H-pyrazolo[3,4-b]pyridin-3-yl]-5-pyrimidinyl}-3-pyrrolidinol (Beispiel XIX, Schritt 3) werden unter Argon in eine frisch hergestellte Lösung aus 0.11 ml Triethylamin und 33 µl DMSO eingebracht und die resultierende Lösung wird auf 0°C gekühlt. Dazu gibt man 73 mg (0.46 mmol) Schwefeltrioxid-Pyridin-Komplex und rührt 1 Stunde bei 0°C nach. Anschließend lässt man auf RT erwärmen und rührt bei dieser Temperatur über Nacht. Die Reaktionslösung wird mit 100 ml destilliertem Wasser versetzt und dreimal mit DCM extrahiert. Die organische Phase wird jeweils einmal mit 1 molarer Salzsäure und destilliertem Wasser gewaschen und das Lösungsmittel unter Vakuum entfernt. Der Rückstand wird mittels präparativer HPLC aufgereinigt. Man erhält 5 mg (5 % d.Th.) des Produktes. LC/MS (Methode C): Rₜ = 3.85 min.
MS (ESIpos): m/z = 389.4 (M+H)⁺
R_{f}= 0.36 (DCM/Ethylacetat 2:1)
¹H-NMR (200 MHz, DMSO-d₆): δ = 2.74 (t, 2H), 3.77 (t, 2H), 3.80 (s, 2H), 5.82 (s, 2H), 7.10-7.48 (m, 5H), 8.42 (s, 2H), 8.64 (d, 1H), 8.84 (d, 1H).

### Beispiel 39

### 1-(2-Fluorbenzyl)-3-[5-(2-methoxy-4-pyridinyl)-2-pyrimidinyl]-1H-pyrazolo[3,4-b]-pyridin

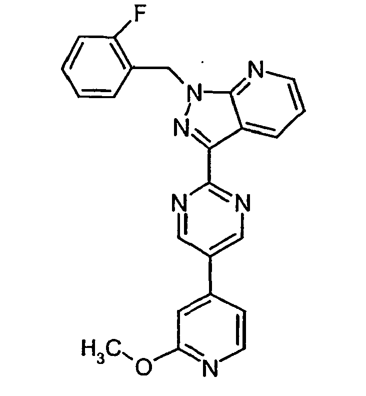

30 mg (0.08 mmol) 1-(2-Fluorbenzyl)-3-[5-(2-fluor-4-pyridinyl)-2-pyrimidinyl]-1H-pyrazolo[3,4-b]pyridin (Beispiel 31) werden in 3 ml Methanol gelöst und mit 32 mg (0.60 mmol) Natiummethanolat versetzt. Die Lösung wird für 3 Tage unter Rückfluss gerührt. Man verdünnt die Lösung mit DCM und extrahiert zweimal mit destilliertem Wasser und einmal mit gesättigter wässriger Natriumchlorid-Lösung. Die organische Phase wird über Magnesiumsulfat getrocknet, filtriert und das Lösungsmittel im Vakuum entfernt. Man erhält 34 mg des Produktes.
LC/MS (Methode C): Rₜ = 3.64 min.
MS (ESIpos): m/z = 413.4 (M+H)⁺
R_{f} = 0.80 (DCM/Methanol 20:1)
¹H-NMR (400 MHz, DMSO-d₆): δ = 3.99 (s, 3H), 5.90 (s, 2H), 7.17 (t, 1H), 7.22-7.33 (m, 2H), 7.47 (dd, 1H), 7.63 (d, 1H), 8.38 (d, 1H), 8.58 (s, 1H), 8.71 (d, 1H), 8.93 (d, 1H), 9.21 (s, 2H).

## Patentansprüche

1. Verbindungen der Formel 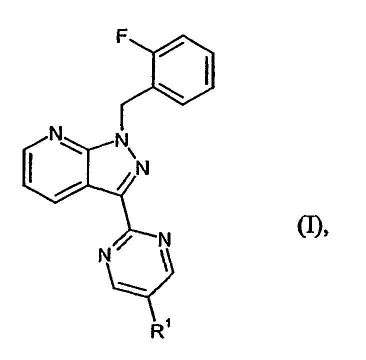
in welcher
R¹ C₆-C₁₀-Aryl oder 5- bis 10-gliedriges Heteroaryl, die gegebenenfalls durch Reste ausgewählt aus der Gruppe Halogen, Cyano, C₁-C₆₋Alkoxy, C₁-C₆-Alkoxycarbonyl, Trifluormethyl, 2,2,2-Trifluorethyl, Trifluormethoxy, C₁-C₄-Alkyl und C₃-C₈-Cycloalkyl substituiert sind, wobei C₁-C₄-Alkyl gegebenenfalls mit Hydroxy substituiert ist,
oder eine Gruppe der Formel 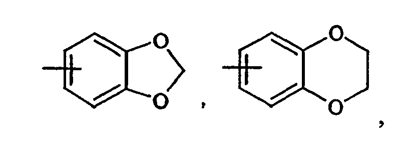
oder
über ein Stickstoffatom gebundenes 4- bis 12-gliedriges Heterocyclyl, das gegebenenfalls durch Reste ausgewählt aus der Gruppe -NHR², Halogen, C₁-C₆-Alkoxycarbonyl, C₁-C₆-Alkoxy, C₁-C₆-Alkyl und Oxo substituiert ist, wobei C₁-C₆-Alkyl gegebenenfalls mit Hydroxy substituiert ist, und
R² C₁-C₄-Alkyl,
oder
C₄-C₈-Cycloalkyl, das in der der Anknüpfungsstelle benachbarten Position durch Oxo substituiert ist und das gegebenenfalls durch C₁₋C₄-Alkyl substituiert ist, bedeuten
und deren Salze, Solvate und/oder Solvate der Salze.

2. Verbindungen nach Anspruch 1, wobei
R¹ Phenyl oder 5- bis 6-gliedriges Heteroaryl, die gegebenenfalls durch Reste ausgewählt aus der Gruppe Fluor, Chlor, Cyano, C₁-C₃₋Alkoxycarbonyl, C₁-C₃-Alkoxy, Trifluormethyl, 2,2,2-Trifluorethyl, Trifluormethoxy, C₁-C₃-Alkyl, und C₃-C₅-Cycloalkyl substituiert sind, wobei C₁-C₃-Alkyl gegebenenfalls mit Hydroxy substituiert ist,
oder ein Gruppe der Formel 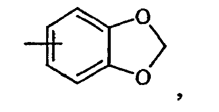
oder
über ein Stickstoffatom gebundenes 4- bis 12-gliedriges Heterocyclyl, das gegebenenfalls durch Reste ausgewählt aus der Gruppe -NHR², Fluor, Chlor, C₁-C₃-Alkyl, C₁-C₃-Alkoxycarbonyl, C₁-C₃-Alkoxy und Oxo substituiert ist, wobei C₁-C₃-Alkyl gegebenenfalls mit Hydroxy substituiert ist und
R² C₁-C₃-Alkyl,
oder
Cyclohexyl, das in der der Anknüpfungsstelle benachbarten Position durch Oxo substituiert ist und das gegebenenfalls durch C₁-C₂-Alkyl substituiert ist, bedeuten
und deren Salze, Solvate und/oder Solvate der Salze.

3. Verbindungen nach Anspruch 1 oder 2, wobei
R¹ Phenyl oder Pyridyl, Pyrazolyl, Isoxazolyl, die gegebenenfalls durch Reste ausgewählt aus der Gruppe Fluor, Chlor, Cyano, Methoxy, Methoxycarbonyl, Ethoxycarbonyl, Trifluormethyl, 2,2,2-Trifluorethyl, Trifluormethoxy, Methyl, Cyclopropyl oder Hydroxymethyl substituiert sind
oder eine Gruppe der Formel 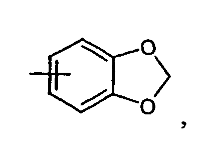
oder
über ein Stickstoffatom gebundenes 4- bis 12-gliedriges Heterocyclyl, das gegebenenfalls durch Reste ausgewählt aus der Gruppe -NHR², Fluor, Chlor, C₁-C₃-Alkyl, Methoxy, Ethoxy, Hydroxymehtyl und Oxo substituiert ist, und
R² Methyl,
oder
Cyclohexyl, das in der der Anknüpfungsstelle benachbarten Position durch Oxo substituiert ist, und das gegebenenfalls durch Methyl substituiert ist, bedeuten
und deren Salze, Solvate und/oder Solvate der Salze.

4. Verfahren zur Herstellung von Verbindungen der Formel (IV) (VT) und (VII), **dadurch gekennzeichnet, dass** man entweder
[A] Verbindungen der Formel 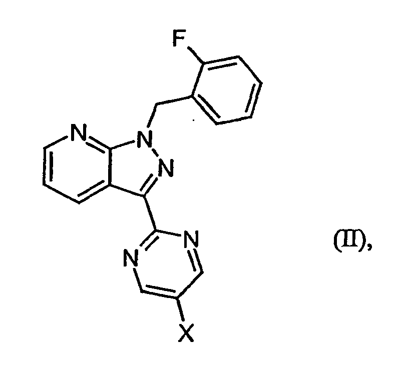 in welcher X Chlor, Brom, Iod, bevorzugt Brom bedeutet,
mit einer Verbindung der Formel
R³-NH-R⁴ (III),
in welcher
R³, R⁴ zusammen mit dem Stickstoffatom, an das sie gebunden sind, 4- bis 12-gliedriges Heterocyclyl, das gegebenenfalls durch Reste ausgewählt aus der Gruppe -NHR², Halogen, C₁-C₆-Alkoxycarbonyl, C₁₋C₆-Alkoxy, C₁-C₆-Alkyl und Oxo substituiert ist, wobei C₁-C₆-Alkyl gegebenenfalls mit -OR⁵ substituiert ist, und R² die oben angegebene Bedeutung hat, R⁵ eine Hydroxy-Schutzgruppe bedeuten in einem inerten Lösungsmittel in Gegenwart einer Base und eines Übergangsmetallkatalysators zu Verbindungen der Formel 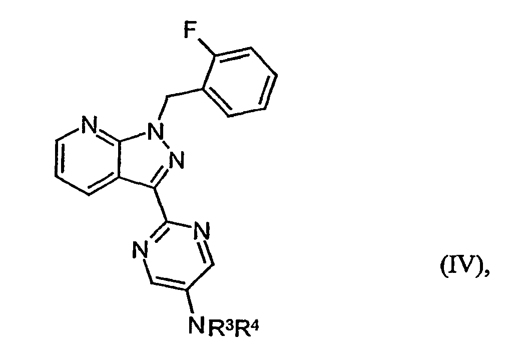
umsetzt,
oder
[B] Verbindungen der Formel (II) mit einer Verbindung der Formel 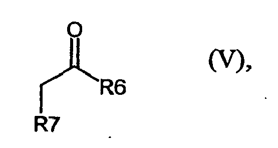
in welcher
R⁶ Cycloalkyl, R⁷ Wasserstoff oder R⁶ und R⁷ zusammen mit der CH₂CO-Gruppe, an der sie gebunden sind, Cycloalkyl, das durch C₁₋C₆-Allcylreste substituiert sein kann, bedeuten in einem inerten Lösungsmittel in Gegenwart einer Base und eines Übergangsmetallkatalysators zu Verbindungen der Formel 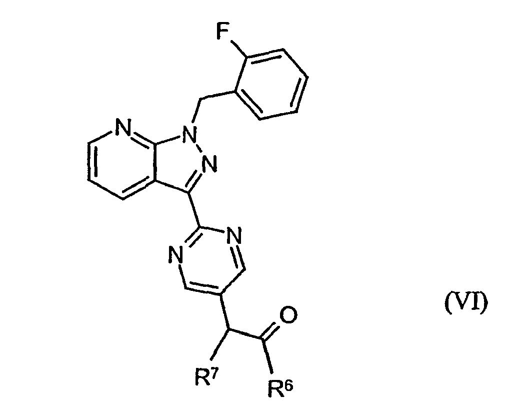
umsetzt,
oder
[C] Verbindungen der Formel (II) mit einer Verbindung der Formel
A-R⁸ (VII),
in welcher
A -B(OR⁹)₂ oder -Sn(C₁-C₆-Alkyl)₃ wobei
R⁹ Wasserstoff, C₁-C₆-Alkyl oder beide Reste zusammen eine -CH₂CH₂- oder -(CH₃)₂C-C(CH₃)₂- Brücke bilden
und
R⁸ C₆-C₁₀-Aryl oder 5- bis 10-gliedriges Heteroaryl, die gegebenenfalls durch Reste ausgewählt aus der Gruppe Halogen, Cyano, C₁-C₆₋Alkoxy, C₁-C₆-Alkoxycarbonyl, Trifluormethyl, 2,2,2-Trifluorethyl,Trifluormethoxy, C₁-C₄-Alkyl und C₃-C₈-Cycloalkyl substituiert sind, wobei C₁-C₄-Alkyl gegebenenfalls mit Hydroxy substituiert ist,
oder eine Gruppe der Formel 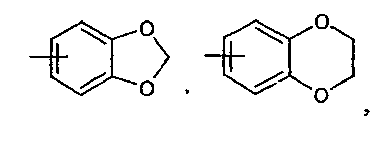
bedeuten, in einem inerten Lösungsmittel in Gegenwart einer Base und eines Übergangsmetallkatalysators zu Verbindungen der Formel 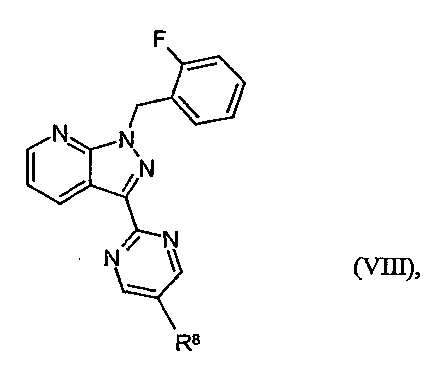 umsetzt,
und die resultierenden Verbindungen der Formeln (IV), (VI) und (VIII) gegebenenfalls mit den entsprechenden (i) Lösungsmitteln und/oder (ii) Basen oder Säuren zu ihren Solvaten, Salzen oder Solvaten der Salze umsetzt.

5. Erfindungsgemäße Verbindungen nach einem der Ansprüche 1 bis 3 zur Behandlung und/oder Prophylaxe von Krankheiten.

6. Arzneimittel enthaltend mindestens eine der Verbindungen nach einem der Ansprüche 1 bis 3 in Zusammenmischung mit mindestens einem pharmazeutisch verträglichen, im wesentlichen nichtgiftigen Träger oder Exzipienten.

7. Verwendung von Verbindungen nach einem der Ansprüche 1 bis 3 zur Herstellung eines Arzneimittels zur Behandlung und/oder Prophylaxe von Krankheiten des Zentralnervensystems.

8. Verwendung von Verbindungen nach einem der Ansprüche 1 bis 3 zur Herstellung eines Arzneimittels zur Behandlung und/oder Prophylaxe von Störungen der Wahrnehmung, Konzentrationsleistung, Lernleistung und/oder Gedächtnisleistung.

9. Arzneimittel nach Anspruch 6 zur Behandlung und/oder Prophylaxe von Krankheiten des Zentralnervensystems.

10. Arzneimittel nach Anspruch 6 zur Behandlung und/oder Prophylaxe von Störungen der Wahrnehmung, Konzentrationsleistung, Lernleistung und/oder Gedächtnisleistung.

## Claims

1. Compounds of the formula 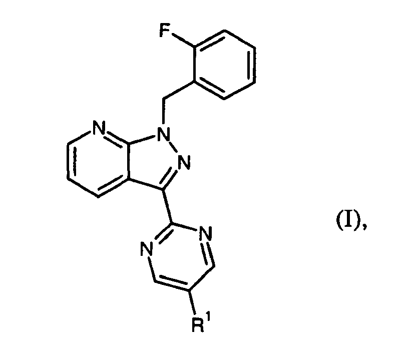 in which
R¹ is C₆-C₁₀-aryl or 5- to 10-membered heteroaryl which are optionally substituted by radicals selected from the group of halogen, cyano, C₁-C₆-alkoxy, C₁-C₆-alkoxycarbonyl, trifluoromethyl, 2,2,2-trifluoroethyl, trifluoromethoxy, C₁-C₄-alkyl and C₃-C₈-cycloalkyl, where C₁-C₄-alkyl is optionally substituted by hydroxy,
or a group of the formula 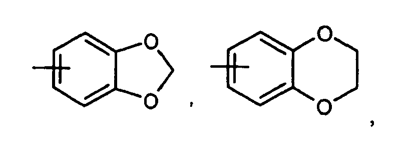
or
4- to 12-membered heterocyclyl which is bonded via a nitrogen atom and which is optionally substituted by radicals selected from the group of -NHR², halogen, C₁-C₆-alkoxycarbonyl, C₁-C₆-alkoxy, C₁-C₆-alkyl and oxo, where C₁-C₆-alkyl is optionally substituted by hydroxy, and
R² is C₁-C₄-alkyl,
or
C₄-C₈-cycloalkyl which is substituted in the position adjacent to the point of attachment by oxo, and which is optionally substituted by C₁-C₄-alkyl,
and the salts, solvates and/or solvates of the salts thereof.

2. Compounds according to Claim 1, wherein
R¹ is phenyl or 5- to 6-membered heteroaryl, which are optionally substituted by radicals selected from the group of fluorine, chlorine, cyano, C₁-C₃-alkoxycarbonyl, C₁-C₃-alkoxy, trifluoromethyl, 2,2,2-trifluoroethyl, trifluoromethoxy, C₁-C₃-alkyl and C₃-C₅-cycloalkyl, where C₁-C₃-alkyl is optionally substituted by hydroxy,
or
a group of the formula 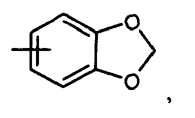
or
4- to 12-membered heterocyclyl which is bonded via a nitrogen atom and which is optionally substituted by radicals selected from the group of -NHR², fluorine, chlorine, C₁-C₃-alkyl, C₁-C₃₋alkoxycarbonyl, C₁-C₃-alkoxy and oxo, where C₁-C₃-alkyl is optionally substituted by hydroxy,
and
R² is C₁-C₃-alkyl,
or
cyclohexyl which is substituted in the position adjacent to the point of attachment by oxo, and which is optionally substituted by C₁-C₂₋alkyl,
and the salts, solvates and/or solvates of the salts thereof.

3. Compounds according to Claim 1 or 2, wherein
R¹ is phenyl or pyridyl, pyrazolyl, isoxazolyl, which are optionally substituted by radicals selected from the group of fluorine, chlorine, cyano, methoxy, methoxycarbonyl, ethoxycarbonyl, trifluoromethyl, 2,2,2-trifluoroethyl, trifluoromethoxy, methyl, cyclopropyl or hydroxymethyl,
or
a group of the formula 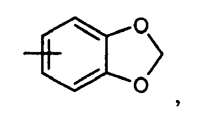
or
4- to 12-membered heterocyclyl which is bonded via a nitrogen atom and which is optionally substituted by radicals selected from the group of -NHR², fluorine, chlorine, C₁-C₃-alkyl, methoxy, ethoxy, hydroxymethyl and oxo, and
R² is methyl,
or
cyclohexyl which is substituted in the position adjacent to the point of attachment by oxo, and which is optionally substituted by methyl, and the salts, solvates and/or solvates of the salts thereof.

4. Process for preparing compounds of the formula (IV), (VI) and (VII), **characterized in that** either
[A] compounds of the formula 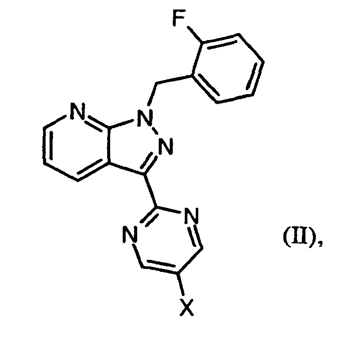
in which X is chlorine, bromine, iodine, preferably bromine,
are reacted with a compound of the formula
R³-NH-R⁴ (III),
in which
R³, R⁴ together with the nitrogen atom to which they are bonded are a 4- to 12-membered heterocyclyl which is optionally substituted by radicals selected from the group of -NHR², halogen, C₁-C₆₋alkoxycarbonyl, C₁-C₆-alkoxy, C₁-C₆-alkyl and oxo, where C₁-C₆₋alkyl is optionally substituted by -OR⁵, and R² has the meaning indicated above, R⁵ is a hydroxy protective group in an inert solvent in the presence of a base and of a transition metal catalyst to give compounds of the formula 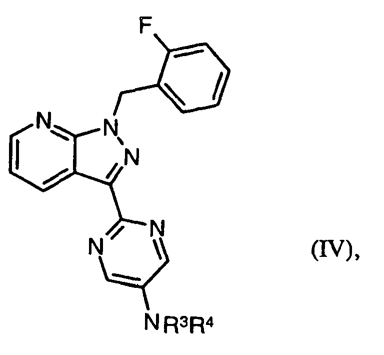
or
[B] compounds of the formula (II) are reacted with a compound of the formula 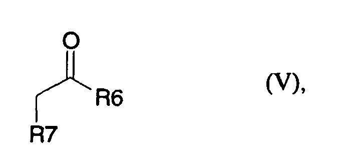 in which
R⁶ is cycloalkyl, R⁷ is hydrogen or R⁶ and R⁷ together with the CH₂CO group to which they are bonded are cycloalkyl which may be substituted by C₁-C₆-alkyl radicals, in an inert solvent in the presence of a base and of a transition metal catalyst to give compounds of the formula 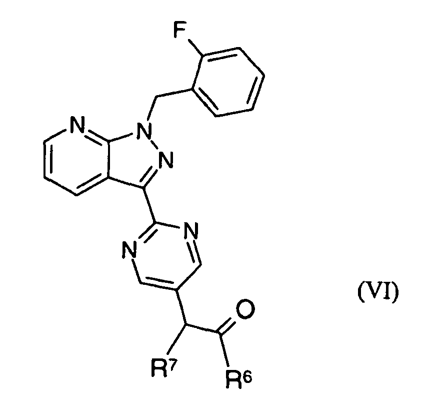
or
[C] compounds of the formula (II) are reacted with a compound of the formula
A-R⁸ (VII),
in which
A is -B(OR⁹)₂ or -Sn(C₁-C₆-alkyl)₃, where
R⁹ is hydrogen, C₁-C₆-alkyl or two radicals together form a -CH₂CH₂- or -(CH₃)₂C-C(CH₃)₂- bridge,
and
R⁸ is C₆-C₁₀-aryl or 5- to 10-membered heteroaryl which are optionally substituted by radicals selected from the group of halogen, cyano, C₁-C₆-alkoxy, C₁-C₆-alkoxycarbonyl, trifluoromethyl, 2,2,2-trifluoroethyl, trifluoromethoxy, C₁-C₄-alkyl and C₃-C₈-cycloalkyl, where C₁-C₄-alkyl is optionally substituted by hydroxy,
or a group of the formula 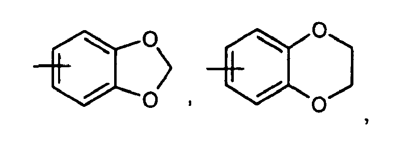
in an inert solvent in the presence of a base and of a transition metal catalyst to give compounds of the formula 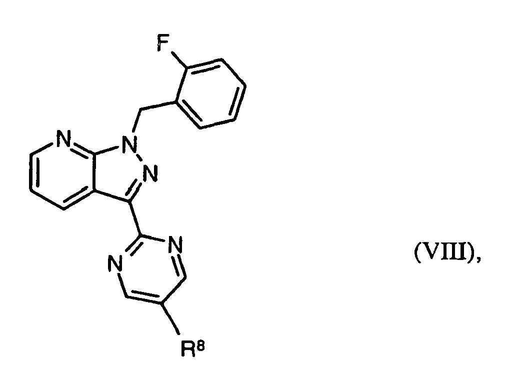 and the resulting compounds of the formula (IV), (VI) and (VIII) are optionally reacted with the appropriate (i) solvents and/or (ii) bases or acids to give the solvates, salts or solvates of the salts thereof.

5. Compounds of the invention according to any of Claims 1 to 3 for the treatment and/or prophylaxis of diseases.

6. Medicament comprising at least one of the compounds according to any of Claims 1 to 3 mixed together with at least one pharmaceutically acceptable, essentially nontoxic carrier or excipient.

7. Use of compounds according to any of Claims 1 to 3 for producing a medicament for the treatment and/or prophylaxis of central nervous system diseases.

8. Use of compounds according to any of Claims 1 to 3 for producing a medicament for the treatment and/or prophylaxis of disorders of perception, concentration, learning and/or memory.

9. Medicament according to Claim 6 for the treatment and/or prophylaxis of central nervous system diseases.

10. Medicament according to Claim 6 for the treatment and/or prophylaxis of disorders of perception, concentration, learning and/or memory.

## Revendications

1. Composés de formule 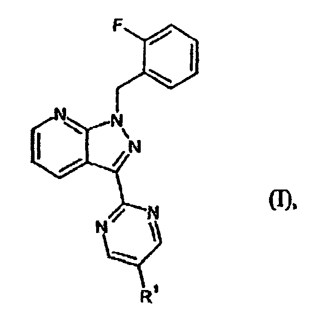 dans laquelle
R¹ est un reste aryle en C₆ à C₁₀ ou un reste hétéroaryle pentagonal à décagonal, qui sont éventuellement substitués par des restes choisis dans le groupe des restes halogéno, cyano, alkoxy en C₁ à C₆, (alkoxy en C₁ à C₆)carbonyle, trifluorométhyle, 2,2,2-trifluoréthyle, trifluorométhoxy, alkyle en C₁ à C₄ et cycloalkyle en C₃ à C₈, le reste alkyle en C₁ à C₄ portant éventuellement un substituant hydroxy,
ou un groupe de formule 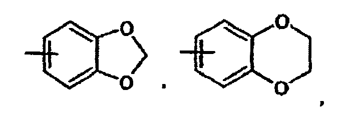
ou bien
un reste hétérocyclyle trétragonal à dodécagonal qui est lié par l'intermédiaire d'un atome d'azote et qui est éventuellement substitué par des restes choisis dans le groupe des restes -NHR², halogéno, (alkoxy en C₁ à C₆) carbonyle, alkoxy en C₁ à C₆, alkyle en C₁ à C₆ et oxo, le reste alkyle en C₁ à C₆ portant éventuellement un substituant hydroxy, et
R² est un reste alkyle en C₁ à C₄
ou bien
un reste cycloalkyle en C₄ à C₈, qui est substitué par un radical oxo dans la position contiguë à la position de liaison et qui est éventuellement substitué par un radical alkyle en C₁ à C₄,
et leurs sels, leurs produits de solvatation et/ou les produits de solvatation de leurs sels.

2. Composés suivant la revendication 1, dans lesquels
R¹ est un reste phényle ou un reste hétéroaryle pentagonal ou hexagonal, qui sont éventuellement substitués par des restes choisis dans le groupe des restes fluoro, chloro, cyano, (alkoxy en C₁ à C₃)carbonyle, alkoxy en C₁ à C₃, trifluorométhyle, 2,2,2-trifluoréthyle, trifluorométhoxy, alkyle en C₁ à C₃ et cycloalkyle en C₃ à C₅, le reste alkyle en C₁ à C₃ portant éventuellement un substituant hydroxy,
ou un groupe de formule 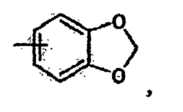
ou bien
un groupe hétérocyclyle tétragonal à dodécagonal, qui est lié par l'intermédiaire de l'atome d'azote et qui est éventuellement substitué par des restes choisis dans le groupe des restes -NHR², fluoro, chloro, alkyle en C₁ à C₃, (alkoxy en C₁ à C₃) carbonyle, alkoxy en C₁ à C₃ et oxo, le reste alkyle en C₁ à C₃ portant éventuellement un substituant hydroxy, et
R² est un reste alkyle en C₁ à C₃,
ou bien
un reste cyclohexyle, qui est substitué par un radical oxo dans la position contiguë à la position de liaison et qui porte éventuellement un substituant alkyle en C₁ ou C₂,
et leurs sels, leurs produits de solvatation et/ou les produits de solvatation de leurs sels.

3. Composés suivant la revendication 1 ou 2, dans lesquels
R¹ représente un reste phényle ou un reste pyridyle, pyrazolyle, isoxazolyle, qui sont éventuellement substitués par des restes choisis dans le groupe des restes fluoro, chloro, cyano, méthoxy, méthoxy-carbonyle, éthoxycarbonyle, trifluorométhyle, 2,2,2-trifluoréthyle, trifluorométhoxy, méthyle, cyclopropyle ou hydroxyméthyle,
ou un groupe de formule 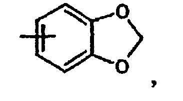 ou bien
un reste hétérocyclyle tétragonal à dodécagonal qui est lié par l'intermédiaire de l'atome d'azote et qui est éventuellement substitué par des restes choisis dans le groupe des restes -NHR², fluoro, chloro, alkyle en C₁ à C₃, méthoxy, éthoxy, hydroxyméthyle et oxo, et
R² est un reste méthyle,
ou bien
un reste cyclohexyle, qui est substitué par un radical oxo dans la position contiguë à la position de liaison et qui est éventuellement substitué par un radical méthyle,
et leurs sels, leurs produits de solvatation et/ou les produits de solvatation de leurs sels.

4. Procédé de production de composés de formules (IV), (VI) et (VII), **caractérisé en ce que**
[A] on fait réagir des composés de formule 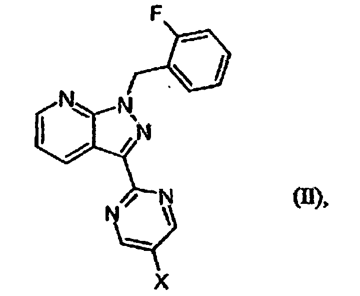
dans laquelle X représente un radical chloro, bromo, iodo, avantageusement un radical bromo, avec un composé de formule
R³-NH-R⁴ (III),
dans laquelle
R³, R⁴ forment conjointement avec l'atome d'azote auquel ils sont liés un reste hétérocyclyle tétragonal à dodécagonal qui est éventuellement substitué par des restes choisis dans le groupe des restes -NHR², halogéno (alkoxy en C₁ à C₆) carbonyle, alkoxy en C₁ à C₆, alkyle en C₁ à C₆ et oxo, le reste alkyle en C₁ à C₆ portant éventuellement un substituant -OR⁵, et R² a la définition indiquée ci-dessus, R⁵ représente un groupe protégeant la fonction hydroxy, dans un solvant inerte en présence d'une base et d'un catalyseur à base de métal de transition, pour obtenir des composés de formule
ou bien
[B] on fait réagir des composés de formule (II) avec un composé de formule 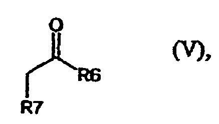
dans laquelle
R⁶ est un reste cycloalkyle, R⁷ représente l'hydrogène, ou bien R⁶ et R⁷ forment, conjointement avec le groupe CH₂CO auquel ils sont liés, un groupe cycloalkyle, qui peut être substitué par des restes alkyle en C₁ à C₆, dans un solvant inerte en présence d'une base et d'un catalyseur à base de métal de transition, pour former des composés de formule 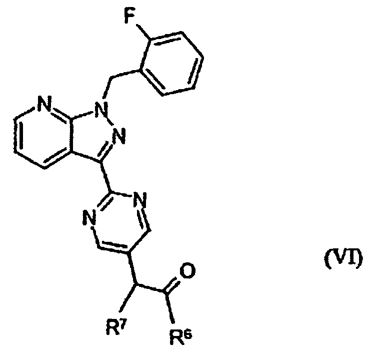
ou bien
[C] on fait réagir des composés de formule (II) avec un composé de formule
A-R⁸ (VII),
dans laquelle
A est un reste -B (OR⁹) ₂ ou -Sn (alkyle en C₁ à C₆)₃. dans lequel
R⁹ représente l'hydrogène, un reste alkyle en C₁ à C₆, ou bien les deux restes forment conjointement un pont -CH₂CH₂- ou -(CH₃)₂C-C(CH₃)₂,
et
R⁸ est un reste aryle en C₆ à C₁₀ ou un reste hétéroaryle pentagonal à décagonal, qui sont éventuellement substitués par des restes choisis dans le groupe des restes halogéno, cyano, alkoxy en C₁ à C₆, (alkoxy en C₁ à C₆)carbonyle, trifluorométhyle, 2,2,2-trifluoréthyl, trifluorométhoxy, alkyle en C₁ à C₄ et cycloalkyle en C₃ à C₈, le reste alkyle en C₁ à C₄ étant éventuellement substitué par un radical hydroxy,
ou un groupe de formule 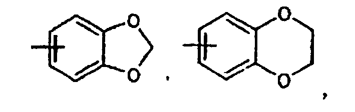 dans un solvant inerte en présence d'une base et d'un catalyseur à base de métal de transition, pour obtenir des composés de formule 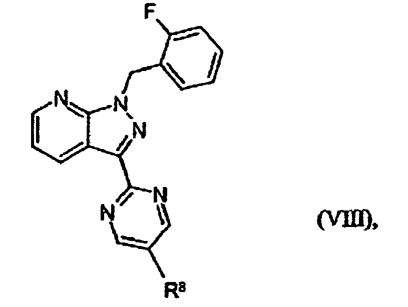 et les composés résultants de formules (IV), (VI) et (VIII) sont éventuellement amenés à réagir avec (i) les solvants convenables et/ou (ii) les bases ou acides convenables pour former leurs produits de solvatation, leurs sels ou des produits de solvatation de leurs sels.

5. Composés conformes à l'invention selon l'une des revendications 1 à 3, destinés au traitement et/ou à la prophylaxie de maladies.

6. Médicament contenant au moins l'un des composés selon l'une des revendications 1 à 3 en mélange avec au moins un support ou excipient principalement non toxique, acceptable du point de vue pharmaceutique.

7. Utilisation de composés suivant l'une des revendications 1 à 3 pour la préparation d'un médicament destiné au traitement et/ou à la prophylaxie de maladies du système nerveux central.

8. Utilisation de composés suivant l'une des revendications 1 à 3 pour la préparation d'un médicament destiné au traitement et/ou à la prophylaxie de troubles de la perception, de la capacité de concentration, de la capacité d'apprentissage et/ou de la capacité de mémoire.

9. Médicament suivant la revendication 6, destiné au traitement et/ou à la prophylaxie de maladies du système nerveux central.

10. Médicament suivant la revendication 6, destiné au traitement et/ou à la prophylaxie de troubles de la perception, de la capacité de concentration, de la capacité d'apprentissage et/ou de la capacité de mémoire.
